(19) Europäisches Patentamt
European Patent Office
Office européen des brevets

(11) Veröffentlichungsnummer: **0 272 533**
**A2**

(12) **EUROPÄISCHE PATENTANMELDUNG**

(21) Anmeldenummer: **87118110.3**

(22) Anmeldetag: **08.12.87**

(51) Int. Cl.4: **C07D 213/69** , C07D 213/64 , C07D 213/70 , C07D 213/71 , C07D 213/73 , C07D 213/74 , C07D 213/76 , C07D 213/75 , C07D 213/77 , C07D 213/84 , C07D 405/12

(30) Priorität: **22.12.86 DE 3643871**
**01.09.87 DE 3729071**

(43) Veröffentlichungstag der Anmeldung:
**29.06.88 Patentblatt 88/26**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Kirsten, Rolf, Dr.**
**Carl-Langhans-Strasse 27**
**D-4019 Monheim(DE)**
Erfinder: **Andree, Roland, Dr.**
**Schillerstrasse 17**
**D-4018 Langenfeld(DE)**
Erfinder: **Busse, Ulrich, Dr.**
**Am Bandenfeld 106**
**D-5657 Haan(DE)**
Erfinder: **Marhold, Albrecht, Dr.**
**Carl-Duisberg-Strasse 329**
**D-5090 Leverkusen 1(DE)**
Erfinder: **Santel, Hans, Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Lürssen, Klaus, Dr.**
**August-Kierspel-Strasse 151**
**D-5060 Bergisch Gladbach 2(DE)**
Erfinder: **Strang, Harry, Dr.**
**Unterdorfstrasse 6A**
**D-4000 Düsseldorf 31(DE)**

(54) **Substituierte Phenoxypyridine.**

(57) Die Erfindung betrifft neue substituierte Phenoxypyridine der Formel (I)

EP 0 272 533 A2

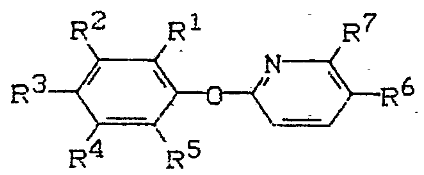

(I)

wobei die Substituenten die in der Beschreibung angegebene Bedeutung haben,
Verfahren sowie neue Zwischenprodukte zu ihrer Herstellung, ihre Verwendung als Herbizide und als Pflanzenwuchsregulatoren.

## Substituierte Phenoxypyridine

Die Erfindung betrifft neue substituierte Phenoxypyridine, Verfahren zu ihrer Herstellung, ihre Verwendung als Herbizide und als Pflanzenwuchsregulatoren sowie neue Zwischenprodukte zu ihrer Herstellung.

Es ist bereits bekannt, daß bestimmte substituierte Phenoxybenzole ("Diphenylether"), wie z. B. 3-(2,4-Dichlor-phenoxy)-6-nitro-benzoesäure-methylester herbizid wirksam sind (vgl. z. B. US-PS 3 652 645 und US-PS 3 776 715). Die Wirkung der bekannten Verbindungen ist jedoch nicht immer zufriedenstellend.

Es wurden nun neue substituierte Phenoxypyridine der Formel (I)

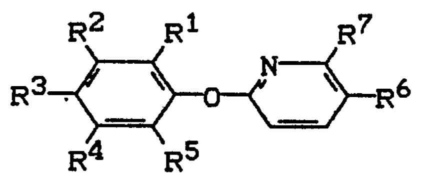

(I)

in welcher

$R^1$ für Wasserstoff, Halogen, Cyano, Nitro oder für einen gegebenenfalls durch Halogen substituierten Rest aus der Reihe Alkyl, Alkoxy, Alkylthio, Alkylsulfinyl und Alkylsulfonyl steht,

$R^2$ für Wasserstoff, Halogen oder Alkyl steht,

$R^3$ für Wasserstoff, Halogen, Cyano, Nitro oder für einen gegebenenfalls durch Halogen substituierten Rest aus der Reihe Alkyl, Alkoxy, Alkylthio, Alkylsulfinyl und Alkylsulfonyl steht,

$R^4$ für Wasserstoff oder Halogen steht und

$R^5$ für Wasserstoff oder Halogen steht

-mit der Maßgabe, daß wenigstens drei der Substituenten $R^1$ bis $R^5$ von Wasserstoff verschieden sind und wenigstens einer der Reste $R^1$ bis $R^5$ von Wasserstoff und Halogen verschieden ist -

in welcher weiter

$R^6$ für Wasserstoff, Cyano, Nitro, Halogen, Carboxy, Carbamoyl oder Alkoxycarbonyl steht und

$R^7$ für Wasserstoff, Halogen, Cyano, Nitro, Formyl, Hydroxy, Trialkylsilyloxy, Alkylaminocarbonyloxy, Dialkoxy(thio)phosphoryloxy, oder für $-O^{\ominus}M^{\oplus}$ steht, wobei

$M^{\oplus}$ für ein Ammoniumion, ein Alkylammoniumion, ein Alkalimetallion oder ein Erdalkalimetallionenäquivalent steht oder

$R^7$ für einen gegebenenfalls durch Halogen, Cyano, Nitro, Carboxy, Carbamoyl, Amino, Phenyl, Alkylcarbonyl, Alkylamino, Dialkylamino, Hydroxy, Alkoxy, Alkylthio, Alkylaminocarbonyl, Alkoxycarbonyl und/oder Alkoxycarbonylalkoxy substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl und Alkylsulfonyl steht oder

$R^7$ für den Rest

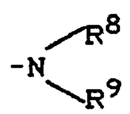

steht, worin

$R^8$ für Wasserstoff oder Alkyl steht und

$R^9$ für Wasserstoff, Hydroxy, Amino, Alkylamino, Dialkylamino, Arylamino, Alkylcarbonylamino, Arylcarbonylamino, Alkylsulfonylamino, gegebenenfalls im Arylteil durch Alkyl substituiertes Arylsulfonylamino, Cyano, Alkyl, Alkoxy, Halogenalkyl, Alkoxycarbonyl, Alkylcarbonyl, Arylcarbonyl, Aryloxycarbonyl, Alkylaminocarbonyl, Arylaminocarbonyl, Alkoxycarbonylalkyl, Aralkyl, Alkylsulfonyl, Arylsulfonyl oder für gegebenenfalls durch Halogen, Cyano, Nitro, Alkyl, Alkoxy, Halogenalkyl und/oder Halogenalkoxy substituiertes Aryl steht, oder

$R^7$ für den Rest $-O-\underset{}{\overset{R^{10}}{\overset{|}{C}H}}-CO-R^{11}$ steht, worin

$R^{10}$ für Wasserstoff oder Alkyl steht und

$R^{11}$ für Halogen, Hydroxy, Amino, Alkylamino, Cyanamino, Dialkylamino, Alkylsulfonylamino, Arylsulfonylamino, Hydroxyamino, Alkoxyamino, Hydrazino, Alkylsulfonylhydrazino, Arylsulfonylhydrazino oder für den

Rest $-Q-R^{12}$ steht, worin
Q für Sauerstoff oder Schwefel steht und
$R^{12}$ für gegebenenfalls durch Halogen substituierte Reste aus der Reihe Alkyl, Alkoxyalkyl, Alkylthioalkyl, Alkylsulfinylalkyl, Alkylsulfonylalkyl, Aryloxyalkyl, Trialkylsilylalkyl, Arylthioalkyl, Aralkoxyalkyl, Alkoxycarbonylalkyl, Alkylaminocarbonylalkyl, Aralkylthioalkyl, Aralkyl, Azolylalkyl oder für ein Ammonium-, Alkylammonium-, Alkalimetall-oder Erdalkalimetall-äquivalent steht, oder
$R^7$ für den Rest

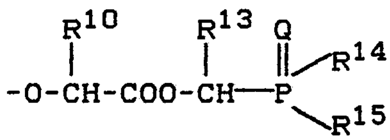

steht, worin
$R^{10}$ für Wasserstoff oder Alkyl steht,
$R^{13}$ für Wasserstoff, Alkyl, Aryl, Furyl, Thienyl oder Pyridyl steht,
$R^{14}$ für Alkyl oder Alkoxy steht,
$R^{15}$ für Alkoxy steht und
Q für Sauerstoff oder Schwefel steht, oder
$R^7$ für den Rest

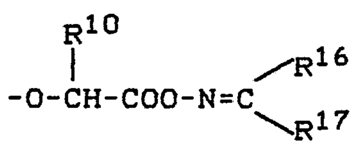

steht, worin
$R^{10}$ für Wasserstoff oder Alkyl steht,
$R^{16}$ für Wasserstoff oder Alkyl steht und
$R^{17}$ für Alkyl oder Aryl steht, oder
$R^7$ für den Rest $-S(O)_n-Y$ steht, worin
n für die Zahlen 0, 1 oder 2 steht und
Y für Alkoxy, Alkenoxy, Alkinoxy, Aralkoxy, Aryloxy oder für Amino, Alkylamino, Alkenylamino, Alkinylamino, Aralkylamino, Arylamino, Dialkylamino, Cyanamino, Guanidino, Hydroxyamino, Alkoxyamino, Hydrazino, Alkylhydrazino, Arylhydrazino, Alkylcarbonylhydrazino, Alkoxycarbonylhydrazino, Alkylsulfonylhydrazino oder Arylsulfonylhydrazino steht, oder
$R^7$ für den Rest

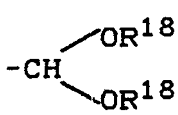

steht, worin die beiden Reste $R^{18}$ einzeln für Alkyl oder zusammen für Alkandiyl ("Alkylen") stehen, oder
$R^7$ für den Rest $-(O)_p-(A)_q-$ 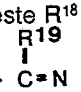 $-R^{20}$ steht, worin,
A für Alkandiyl (Alkylen) steht,
p und q jeweils für die Zahlen 0 oder 1 stehen,
$R^{19}$ für Wasserstoff, Alkyl, Alkoxy oder Alkylamino steht und
$R^{20}$ für Wasserstoff, Alkyl, Aryl, Hydroxy, für gegebenenfalls durch Alkoxycarbonyl substituiertes Alkoxy, für Alkenyloxy, Alkinyloxy oder Aralkoxy steht, oder
$R^7$ für den Rest

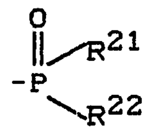

steht, worin

$R^{21}$ für Alkyl oder Alkoxy steht und

$R^{22}$ für Alkoxy steht, oder

$R^7$ für den Rest $-(CH_2)_m-Z$ oder für den Rest

$-O-\overset{R^{10}}{\overset{|}{C}}H-COO-(CH_2)_m-Z$ steht, worin

m für die Zahlen 0, 1 oder 2 steht,

$R^{10}$ für Wasserstoff oder Alkyl steht, und

Z für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl, Pyrimidinyl und Pyridazinyl steht,

gefunden.

Soweit die neuen substituierten Phenoxypyridine der Formel (I) Substituenten mit asymmetrischen Kohlenstoffatomen enthalten, betrifft die Erfindung sowohl die einzelnen möglichen Isomeren als auch Gemische dieser Isomeren.

Die Maßgabe in der Definition von $R^5$ wurde wegen vorbekannter Verbindungen eingeführt (vgl. EP-A 72.529 und EP-A 109.751, US-P 3 576 616 und US-P 4 493 730).

Weiter wurde gefunden, daß man substituierte Phenoxypyridine der Formel (I) erhält, wenn man

(a) Phenole der Formel (II)

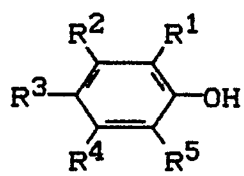

(II)

in welcher

$R^1$, $R^2$, $R^3$, $R^4$ und $R^5$ die oben angegebenen Bedeutungen haben,

oder Alkalimetallsalze von Phenolen der Formel (II) mit Halogenpyridinen der Formel (III)

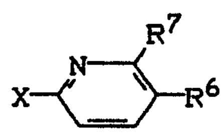

(III)

in welcher

$R^6$ und $R^7$ die oben angegebenen Bedeutungen haben und

X für Halogen steht,

gegebenenfalls in Gegenwart von Säureakzeptoren und gegebenenfalls in Gegenwart von Verdünnungsmitteln umsetzt

und gegebenenfalls an den so erhaltenen Verbindungen der Formel (I) weitere Derivatisierungen in dem durch die obige Substituentendefinition vorgegebenen Rahmen nach üblichen Methoden gemäß den Verfahren (b) bis (j) durchführt:

So erhält man Verbindungen der Formel (I)

(b) für den Fall, daß $R^7$ für Hydroxy steht und die Reste $R^1$ bis $R^6$ die oben angegebenen Bedeutungen haben, wenn man Verbindungen der Formel (I), in welcher $R^7$ für Amino steht und die Reste $R^1$ bis $R^6$ die oben angegebenen Bedeutungen haben, mit einem Alkalinitrit in Gegenwart einer wäßrigen Mineralsäure umsetzt, oder

(c) für den Fall, daß $R^7$ für Carboxyalkoxy ($-O-CHR^{10}-COOH$) steht und die Reste $R^1$ bis $R^6$ die oben angegebene Bedeutung haben, wenn man Verbindungen der Formel (I), in welcher $R^7$ für Alkoxycarbonylalkoxy steht und die Reste $R^1$ bis $R^6$ die oben angegebenen Bedeutungen haben, mit einem Alkalihydroxid in Gegenwart von Wasser und gegebenenfalls in Gegenwart eines organischen Lösungsmittels umsetzt und dann - gegebenenfalls nach Einengen - mit einer Mineralsäure ansäuert, oder

(d) für den Fall, daß $R^7$ für Chlorcarbonylalkoxy steht und die Reste $R^1$ bis $R^6$ die oben angegebene Bedeutung haben, wenn man Verbindungen der Formel (I), in welcher $R^7$ für carboxyalkoxy steht und die Reste $R^1$ bis $R^6$ die oben angegebenen Bedeutungen haben, mit einem Halogenierungsmittel gegebenenfalls in Gegenwart eines Verdünnungsmittels umsetzt, oder

(e) für den Fall, daß $R^7$ für den Rest

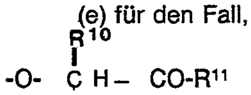

oder für den Rest 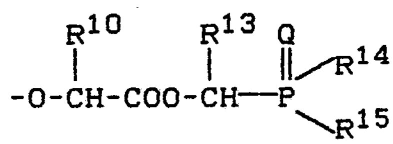

oder für den Rest 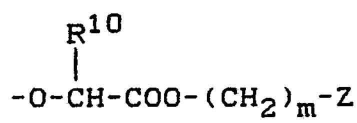

oder für den Rest 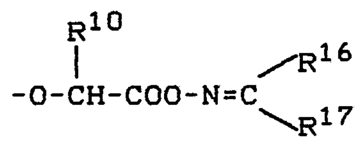

steht, worin $R^{10}$, $R^{11}$, $R^{13}$, $R^{14}$, $R^{15}$, $R^{16}$, $R^{17}$, Z, Q und m die oben angegebenen Bedeutungen haben und weiter die Reste $R^1$ bis $R^6$ die oben angegebenen Bedeutungen haben,
wenn man Verbindungen der Formel (I), in welcher $R^7$ für Chlorcarbonylalkoxy steht und die Reste $R^1$ bis $R^6$ die oben angegebenen Bedeutungen haben - Chlorcarbonylalkoxy steht in diesem Fall für den

Rest 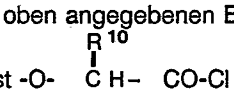 -
mit Verbindungen der Formel $H-R^{11}$

bzw. der Formel 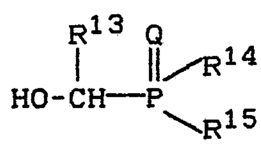

bzw. der Formel $HO-(CH_2)_m-Z$

bzw. der Formel 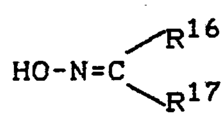

worin $R^{11}$, $R^{13}$, $R^{14}$, $R^{15}$, $R^{16}$, $R^{17}$, Z, Q und m die oben angegebenen Bedeutungen haben, gegebenenfalls in Gegenwart eines Säureakzeptors und gegebenenfalls in Gegenwart eines Verdünnungsmittels umsetzt, oder

(f) für den Fall, daß $R^7$ für den Rest

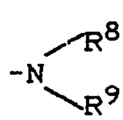

steht,

worin

$R^8$ die oben angegebene Bedeutung hat und

$R^9$ für Alkoxycarbonyl, Alkylcarbonyl, Arylcarbonyl, Aryloxycarbonyl, Alkylaminocarbonyl, Arylaminocarbonyl, Alkylsulfonyl oder Arylsulfonyl steht

und die Reste $R^1$ bis $R^6$ die oben angegebenen Bedeutungen haben,

wenn man Verbindungen der Formel (I), in welcher $R^7$ für den Rest -NH-$R^8$ steht, worin $R^8$ die oben angegebene Bedeutung hat, und die Reste $R^1$ bis $R^6$ die oben angegebenen Bedeutungen haben,

mit Alkoxycarbonyl-, Alkylcarbonyl-, Arylcarbonyl-, Aryloxycarbonyl-, Alkylsulfonyl-oder Arylsulfonylhalogeniden (insbesondere -chloriden) bzw. den entsprechenden Anhydriden bzw. mit Alkyl-oder Arylisocyanaten, gegebenenfalls in Gegenwart eines Säureakzeptors und gegebenenfalls in Gegenwart eines Verdünnungsmittels umsetzt, oder

(g) für den Fall, daß $R^7$ für Alkylsulfinyl oder Alkylsulfonyl steht und die Reste $R^1$ bis $R^6$ die oben angegebenen Bedeutungen haben,

wenn man Verbindungen der Formel (I), in welcher $R^7$ für Alkylthio steht und die Reste $R^1$ bis $R^6$ die oben angegebenen Bedeutungen haben,

mit einem Oxidationsmittel, gegebenenfalls in Gegenwart eines Katalysators und gegebenenfalls in Gegenwart eines Verdünnungsmittels umsetzt, oder

(h) für den Fall, daß $R^7$ für Alkoxy steht und die Reste $R^1$ bis $R^6$ die oben angegebenen Bedeutungen haben,

wenn man Verbindungen der Formel (I), in welcher $R^7$ für Halogen steht und die Reste $R^1$ bis $R^6$ die oben angegebenen Bedeutungen Haben,

mit Alkanolen und/oder Alkalimetallsalzen von Alkanolen umsetzt, oder

(i) für den Fall, daß $R^7$ für Halogen steht und die Reste $R^1$ bis $R^6$ die oben angegebenen Bedeutungen haben,

wenn man Verbindungen der Formel (I), in welcher $R^7$ für Amino steht und die Reste $R^1$ bis $R^6$ die oben angegebenen Bedeutungen haben,

mit einem Alkalimetallnitrit und einem Hydrogen-oder Metall-halogenid bzw. Metallhalogenid-Komplex, in Gegenwart einer Säure, gegebenenfalls in Gegenwart eines Katalysators und in Gegenwart eines Verdünnungsmittels umsetzt, oder

(j) für den Fall, daß $R^7$ für den Rest $-O-\overset{R^{10}}{\overset{|}{C}H}-COOR^{12}$ steht, worin

$R^{10}$ für Wasserstoff oder Alkyl steht und

$R^{12}$ für Trialkylsilylalkyl steht,

und die Reste $R^1$ bis $R^6$ die oben angegebenen Bedeutungen haben,

wenn man Verbindungen der Formel (I), in welcher

$R^7$ für den Rest $-O-\overset{R^{10}}{\overset{|}{C}H}-COOH$ steht, worin

$R^{10}$ die oben angegebenen Bedeutungen hat,

mit Trialkylsilylalkylhalogeniden, gegebenenfalls in Gegenwart eines Säureakzeptors und gegebenenfalls in Gegenwart eines Verdünnungsmittels umsetzt.

Schließlich wurde gefunden, daß die neuen substituierten Phenoxypyridine der Formel (I) hervorragende herbizide bzw. pflanzenwuchsregulierende Eigenschaften aufweisen.

Überraschenderweise sind die erfindungsgemäßen substituierten Phenoxypyridine der Formel (I) gegen einige wichtige Unkräuter wesentlich stärker wirksam und zeigen wesentlich bessere selektive Eigenschaften als 3-(2,4-Dichlor-phenoxy)-6-nitro-benzoesäuremethylester, welches ein strukturell ähnlicher vorbekannter Wirkstoff gleicher Wirkungsrichtung ist.

Die Kohlenwasserstoffketten in den einzelnen Resten wie beispielsweise Alkyl, Alkoxy, Alkylthio, Alkoxycarbonyl, Alkenyl oder Halogenalkyl sind jeweils geradkettig oder verzweigt. Die Substitution kann jeweils einfach oder mehrfach, gleich oder verschieden erfolgen.

Die Erfindung betrifft vorzugsweise Verbindungen der Formel (I), in welcher

$R^1$ für Wasserstoff, Fluor, Chlor, Brom, Cyano, Nitro oder für einen gegebenenfalls durch Fluor und/oder Chlor substituierten Rest aus der Reihe $C_1$-$C_2$-Alkyl, $C_1$-$C_2$-Alkoxy, $C_1$-$C_2$-Alkylthio, $C_1$-$C_2$-Alkylsulfinyl und $C_1$-$C_2$-Alkylsulfonyl steht,

$R^2$ für Wasserstoff, Fluor, Chlor, Brom oder $C_1$-$C_2$-Alkyl steht,

$R^3$ für Wasserstoff, Fluor, Chlor Brom, Cyano, Nitro oder für einen gegebenenfalls durch Fluor und/oder Chlor substituierten Rest aus der Reihe $C_1$-$C_4$-Alkyl, $C_1$-$C_4$-Alkoxy, $C_1$-$C_4$-Alkylthio, $C_1$-$C_4$-Alkylsulfinyl und $C_1$-$C_4$-Alkylsulfonyl steht,

$R^4$ für Wasserstoff, Fluor, Chlor oder Brom steht und

$R^5$ für Wasserstoff, Fluor, Chlor oder Brom steht

-mit der Maßgabe, daß wenigstens drei der Substituenten $R^1$ bis $R^5$ von Wasserstoff verschieden sind und wenigstens einer der Reste $R^1$ bis $R^5$ von Wasserstoff, Fluor, Chlor und Brom verschieden ist -

in welcher weiter

$R^6$ für Wasserstoff, Cyano, Nitro, Fluor, Chlor, Brom, Carboxy, Carbamoyl oder $C_1$-$C_4$-Alkoxycarbonyl steht und

$R^7$ für Wasserstoff, Fluor, Chlor, Brom, Cyano, Nitro, Formyl, Hydroxy, Trimethylsilyloxy, $C_1$-$C_4$-Alkylaminocarbonyloxy, Di-($C_1$-$C_4$-alkoxy)-(thio)phosphoryloxy oder für $-O^{\ominus}M^{\oplus}$ steht, wobei

$M^{\oplus}$ für ein Ammoniumion, ein $C_1$-$C_4$-Alkylammoniumion, ein Natriumion, ein Kaliumion oder ein Calciumionenäquivalent steht, oder

$R^7$ für gegebenenfalls durch Fluor, Chlor, Brom, Cyano, Nitro, Carboxy, Carbamoyl, Amino, Phenyl, $C_1$-$C_4$-Alkyl-carbonyl, $C_1$-$C_4$-Alkylamino, Di-($C_1$-$C_4$-alkyl)-amino, Hydroxy, $C_1$-$C_4$-Alkoxy, $C_1$-$C_4$-Alkylthio, $C_1$-$C_4$-Alkylaminocarbonyl, $C_1$-$C_4$-Alkoxy-carbonyl und/oder $C_1$-$C_4$-Alkoxy-carbonyl-$C_1$-$C_4$-alkoxy substituiertes $C_1$-$C_6$-Alkyl, für gegebenenfalls durch Fluor, Chlor, Brom, Cyano, Nitro, Carboxy, Carbamoyl und/oder $C_1$-$C_4$-Alkoxycarbonyl substituiertes $C_2$-$C_6$-Alkenyl, für gegebenenfalls durch Fluor, Chlor, Brom, Cyano und/oder $C_1$-$C_4$-Alkoxycarbonyl substituiertes $C_2$-$C_6$-Alkinyl, für gegebenenfalls durch Fluor, Chlor, Cyano, Di-($C_1$-$C_4$-alkyl)-amino, Hydroxy, $C_1$-$C_4$-Alkylcarbonyl, $C_1$-$C_4$-Alkoxy und/oder $C_1$-$C_4$-Alkylthio substituiertes $C_1$-$C_4$-Alkoxy, für gegebenenfalls durch Fluor, Chlor, Cyano und/oder $C_1$-$C_4$-Alkoxy-carbonyl substituiertes $C_2$-$C_4$-Alkenyloxy, für gegebenenfalls durch Fluor, Chlor, Brom, Cyano und/oder $C_1$-$C_4$-Alkoxy-carbonyl substituiertes $C_2$-$C_4$-Alkinyloxy, für gegebenenfalls durch Fluor, Chlor, Brom, Cyano, Carboxy, Carbamoyl, $C_1$-$C_4$-Alkoxy, $C_1$-$C_4$-Alkylthio und/oder $C_1$-$C_4$-Alkoxy-carbonyl substituiertes $C_1$-$C_4$-Alkylthio, für gegebenenfalls durch Fluor, Chlor und/oder Brom substituiertes $C_2$-$C_4$-Alkenylthio, für jeweils gegebenenfalls durch Fluor, Chlor oder Brom substituiertes $C_2$-$C_4$-Alkinylthio, $C_1$-$C_4$-Alkylsulfinyl oder $C_1$-$C_4$-Alkylsulfonyl steht

oder

$R^7$ für den Rest

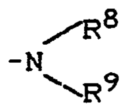

steht, worin

$R^8$ für Wasserstoff oder $C_1$-$C_4$-Alkyl steht und

$R^9$ für Wasserstoff, Hydroxy, Amino, $C_1$-$C_6$-Alkylamino, Dimethylamino, Phenylamino, $C_1$-$C_4$-Alkyl-carbonylamino, Phenylcarbonylamino, $C_1$-$C_4$-Alkylsulfonylamino, Phenylsulfonylamino, 4-Methyl-phenylsulfonylamino, Cyano, $C_1$-$C_4$-Alkyl, $C_1$-$C_4$-Alkoxy, $C_1$-$C_4$-Fluoralkyl, $C_1$-$C_4$-Alkoxycarbonyl, $C_1$-$C_4$-Alkyl-carbonyl, Benzoyl, Phenoxycarbonyl, $C_1$-$C_4$-Alkylamino-carbonyl, Phenylaminocarbonyl, $C_1$-$C_4$-Alkoxy-carbonyl-$C_1$-$C_2$-alkyl, Benzyl, $C_1$-$C_4$-Alkylsulfonyl, Phenylsulfonyl oder für gegebenenfalls durch Fluor, Chlor, Brom, Cyano, Nitro, $C_1$-$C_4$-Alkyl, $C_1$-$C_4$-Alkoxy, $C_1$-$C_4$-Halogenalkyl und/oder $C_1$-$C_4$-Halogenalkoxy substituiertes Phenyl steht,

oder

$R^7$ für den Rest $-O-\overset{R^{10}}{\overset{|}{C}H}-CO-R^{11}$ steht, worin

$R^{10}$ für Wasserstoff oder $C_1$-$C_4$-Alkyl steht und

$R^{11}$ für Chlor, Hydroxy, Amino, $C_1$-$C_4$-Alkylamino, Cyanamino, Di-($C_1$-$C_4$-alkyl)-amino, $C_1$-$C_4$-Alkylsulfonylamino, Phenylsulfonylamino, Hydroxyamino, $C_1$-$C_4$-Alkoxyamino, Hydrazino, $C_1$-$C_4$-Alkylsulfonyl-hydrazino, Phenylsulfonylhydrazino oder für den Rest $-Q-R^{12}$ steht, worin

Q für Sauerstoff oder Schwefel steht und

$R^{12}$ für gegebenenfalls durch Fluor und/oder Chlor substituierte Reste aus der Reihe $C_1$-$C_6$-Alkyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_4$-alkyl, Trimethylsilyl-$C_1$-$C_4$-alkyl, Phenylthio-$C_1$-$C_4$-alkyl, Benzyloxy-$C_1$-$C_4$-alkyl, $C_1$-$C_4$-Alkoxycarbonyl-$C_1$-$C_4$-alkyl, $C_1$-$C_4$-Alkylamino-carbonyl-$C_1$-$C_4$-alkyl, Benzylthio-$C_1$-$C_4$-alkyl, Benzyl, Phenethyl, Pyrazolyl-$C_1$-$C_4$-alkyl, Imidazolyl-$C_1$-$C_4$-alkyl, Triazolyl-$C_1$-$C_4$-alkyl oder für ein Ammonium-, ein $C_1$-$C_4$-Alkylammonium-, ein Natrium-Kalium-oder Calcium-Äquivalent steht,

oder

$R^7$ für den Rest

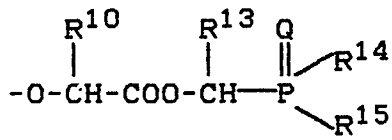

steht, worin
$R^{10}$ für Wasserstoff oder $C_1$-$C_4$-Alkyl steht,
$R^{13}$ für Wasserstoff, $C_1$-$C_4$-Alkyl, Phenyl, Furyl, Thienyl oder Pyridyl steht,
$R^{14}$ für $C_1$-$C_4$-Alkyl oder $C_1$-$C_4$-Alkoxy steht,
$R^{15}$ für $C_1$-$C_4$-Alkoxy steht und
Q für Sauerstoff oder Schwefel steht,
oder
$R^7$ für den Rest

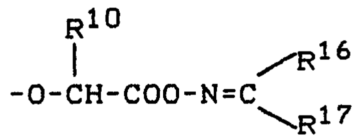

steht, worin
$R^{10}$ für Wasserstoff oder $C_1$-$C_4$-Alkyl steht,
$R^{16}$ für Wasserstoff oder $C_1$-$C_4$-Alkyl steht und
$R^{17}$ für $C_1$-$C_4$-Alkyl oder Phenyl steht,
oder
$R^7$ für den Rest $-S(O)_n-Y$ steht, worin
n für die Zahlen 0, 1 oder 2 steht und
Y für $C_1$-$C_6$-Alkoxyl, $C_2$-$C_6$-Alkenoxy, $C_2$-$C_6$-Alkinoxy, Benzyloxy, Phenoxy oder für Amino, $C_1$-$C_6$-Alkylamino, $C_2$-$C_6$-Alkenylamino, $C_2$-$C_6$-Alkinyl amino, Benzylamino, Phenylamino, Di-($C_1$-$C_4$-alkyl)-amino, Cyanamino, Guanidino, Hydroxyamino, $C_1$-$C_6$-Alkoxyamino, Hydrazino, $C_1$-$C_4$-Alkylhydrazino, Phenylhydrazino, $C_1$-$C_4$-Alkylcarbonylhydrazino, $C_1$-$C_4$-Alkoxycarbonylhydrazino, $C_1$-$C_4$-Alkylsulfonylhydrazino oder Phenylsulfonylhydrazino steht,
oder
$R^7$ für den Rest

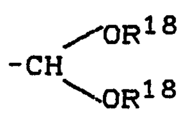

steht, worin die beiden Reste $R^{18}$ einzeln für $C_1$-$C_4$-Alkyl oder zusammen für $C_2$-$C_3$-Alkandiyl stehen,
oder
$R^7$ für den Rest $-(O)_p-(A)_q-$ 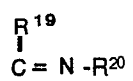 steht, worin
$R^{19}$ für Wasserstoff, $C_1$-$C_4$-Alkyl, $C_1$-$C_4$-Alkoxy oder $C_1$-$C_4$-Alkylamino steht und
$R^{20}$ für Wasserstoff, $C_1$-$C_4$-Alkyl, Phenyl, Hydroxy, für gegebenenfalls durch $C_1$-$C_4$-Alkoxy-carbonyl substituiertes $C_1$-$C_4$-Alkoxy, für Allyloxy, Propargyloxy oder Benzyloxy steht,
A für $C_1$-$C_4$-Alkandiyl steht und
p und q jeweils für die Zahlen 0 oder 1 stehen,
oder
$R^7$ für den Rest

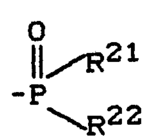

steht, worin
$R^{21}$ für $C_1$-$C_4$-Alkyl oder $C_1$-$C_4$-Alkoxy steht und
$R^{22}$ für $C_1$-$C_4$-Alkoxy steht,
oder
$R^7$ für den Rest $-(CH_2)_m$-Z oder für den Rest

$-O-\overset{R^{10}}{\overset{|}{C}}H-COO-(CH_2)_m-Z$ steht, worin
m für die Zahlen 0, 1 oder 2 steht,
Z für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl, Pyrimidinyl und Pyridazinyl steht und
$R^{10}$ für Wasserstoff oder $C_1$-$C_4$-Alkyl steht.

Die Erfindung betrifft insbesondere Verbindungen der Formel (I), in welcher
$R^1$ für Wasserstoff, Fluor, Chlor, Cyano oder Trifluormethyl steht,
$R^2$ für Wasserstoff, Fluor oder Chlor steht,
$R^3$ für Fluor, Chlor, Cyano, Methyl, Ethyl, n-oder i-Propyl, n-, i-, s-oder t-Butyl, Methoxy, Methylthio, Trifluormethyl, Trifluormethoxy, Trifluormethylthio, Trifluormethylsulfinyl oder Trifluormethylsulfonyl steht,
$R^4$ für Wasserstoff, Fluor oder Chlor steht und
$R^5$ für Wasserstoff, Fluor oder Chlor steht
-mit der Maßgabe, daß wenigstens drei der Substituen $R^1$ bis $R^5$ von Wasserstoff verschieden sind und wenigstens einer der Reste $R^1$ bis $R^5$ von Wasser-stoff, Fluor und Chlor verschieden ist -
in welcher weiter
$R^6$ für Wasserstoff, Cyano, Nitro oder Chlor steht und
$R^7$ für Wasserstoff, Chlor, Cyano, Nitro, Hydroxy, Di-($C_1$-$C_2$-alkoxy)-(thio)phosphoryloxy oder für $O^{\ominus}M^{\oplus}$ steht, wobei
$M^{\oplus}$ für ein Ammoniumion, ein $C_1$-$C_3$-Alkylammoniumion, ein Natriumion oder ein Kaliumion steht,
oder
$R^7$ für gegebenenfalls durch Fluor, Chlor, Cyano, Carboxy, Carbamoyl, Acetyl, Phenyl, Amino, $C_1$-$C_3$-Alkylamino, Di-($C_1$-$C_3$-alkyl)-amino, Hydroxy, $C_1$-$C_3$-Alkoxy, $C_1$-$C_3$-Alkylthio, $C_1$-$C_3$-Alkoxycarbonyl und/oder $C_1$-$C_3$-Alkoxy-carbonyl-$C_1$-$C_3$-alkoxy substituiertes $C_1$-$C_3$-Alkyl, für gegebenenfalls durch Fluor, Chlor, Cyano, Di-($C_1$-$C_3$-alkyl)-amino, Hydroxy, $C_1$-$C_3$-Alkylcarbonyl, $C_1$-$C_3$-Alkoxy und/oder $C_1$-$C_3$-Alkylthio substituiertes $C_1$-$C_3$-Alkoxy, für $C_3$-$C_4$-Alkenyloxy, für $C_3$-$C_4$-Alkinyloxy, für gegebenenfalls durch Fluor, Chlor, Cyano, Carboxy, Carbamoyl, $C_1$-$C_3$-Alkoxy, $C_1$-$C_3$-Alkylthio oder $C_1$-$C_3$-Alkoxy-carbonyl substituiertes $C_1$-$C_3$-Alkylthio, für jeweils gegebenenfalls durch Fluor und/oder Chlor substituiertes $C_1$-$C_3$-Alkylsulfinyl oder $C_1$-$C_3$-Alkylsulfonyl, Hydrazino, Phenylhydrazino oder Methoxycarbonylhydrazino steht,
oder
$R^7$ für den Rest

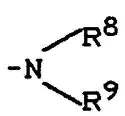 steht, worin

$R^8$ für Wasserstoff oder $C_1$-$C_3$-Alkyl steht und
$R^9$ für Wasserstoff, Hydroxy, Amino, Dimethylamino, Phenylamino, Acetylamino, Benzoylamino, Methylsulfonylamino, Phenylsulfonylamino, Cyano, $C_1$-$C_3$-Alkyl, $C_1$-$C_3$-Alkoxy, $C_1$-$C_3$-Alkoxycarbonyl, $C_1$-$C_3$-Alkylcarbonyl, Benzoyl, Phenoxycarbonyl, $C_1$-$C_3$-Alkylamino-carbonyl, Phenylamino-carbonyl, $C_1$-$C_3$-Alkoxy-carbonyl-$C_1$-$C_2$-Alkyl, Benzyl, $C_1$-$C_3$-Alkylsulfonyl, Phenylsulfonyl oder für gegebenenfalls durch Fluor, Chlor, Brom, Cyano, Nitro, Methyl, Methoxy, Trifluormethyl und/oder Trifluormethoxy substituiertes Phenyl steht,
oder
$R^7$ für den Rest $-O-\overset{R^{10}}{\overset{|}{C}}H-CO-R^{11}$ steht, worin
$R^{10}$ für Wasserstoff oder Methyl steht und
$R^{11}$ für Chlor, Hydroxy, Amino, $C_1$-$C_3$-Alkylamino, Cyanamino, Di-($C_1$-$C_3$-alkyl)-amino, $C_1$-$C_3$-Alkylsulfonylamino, Phenylsulfonylamino, Hydroxyamino, $C_1$-$C_3$-Alkoxyamino, Hydrazino, $C_1$-$C_3$-Alkylsulfonylhydrazino, Phenylsulfonylhydrazino oder für den Rest $-Q-R^{12}$ steht, worin
Q für Sauerstoff oder Schwefel steht und

$R^{12}$ für $C_1$-$C_4$-Alkyl, $C_1$-$C_2$-Alkoxy-ethyl, $C_1$-$C_2$-Alkylthio-ethyl, Trimethylsilylmethyl, Benzyloxyethyl, $C_1$-$C_3$-Alkoxyl-Carbonyl-$C_1$-$C_2$-alkyl, Benzylthioethyl, Benzyl, Pyrazol-$C_1$-$C_2$-alkyl oder für ein Ammonium-, ein $C_1$-$C_3$-Alkylammoniumion, ein Natrium-oder ein Kalium-äquivalent oder für $C_1$-$C_2$-Alkylamino-carbonyl-$C_1$-$C_2$-alkyl steht,

oder

$R^7$ für den Rest

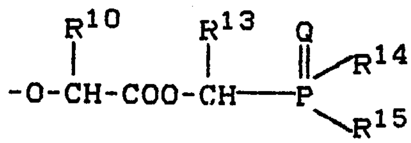

steht, worin

$R^{10}$ für Wasserstoff oder Methyl steht,

$R^{13}$ für Wasserstoff, Methyl, Phenyl, Furyl, Thienyl oder Pyridyl steht,

$R^{14}$ für $C_1$-$C_2$-Alkoxy steht,

$R^{15}$ für $C_1$-$C_2$-Alkoxy steht und

Q für Sauerstoff oder Schwefel steht,

oder

$R^7$ für den Rest $-S(O)_n-Y$ steht, worin

n für die Zahlen 0, 1 oder 2 steht und

Y für $C_1$-$C_4$-Alkyl, $C_1$-$C_2$-Alkoxy-carbonyl-$C_1$-$C_2$-alkyl oder Benzyl steht oder

$R^7$ für den Rest $-(CH_2)_m-Z$ oder

für den Rest -O- 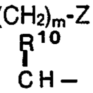 COO-$(CH_2)_m$-Z steht, worin

m für die Zahlen 0, 1 oder 2 steht,

Z für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl und Dioxolanyl steht und

$R^{10}$ für Wasserstoff oder Methyl steht, oder

$R^7$ für den Rest -O-$CH_2$- 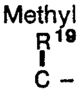 – C = N-$R^{20}$ steht, worin

$R^{19}$ für Wasserstoff, Methyl oder Ethyl steht und

$R^{20}$ für Wasserstoff, Hydroxy, Methyl, Ethyl oder $C_1$-$C_2$-Alkoxycarbonyl-$C_1$-$C_2$-alkyl steht.

Ganz besonders bevorzugt sind Verbindungen der Formel (I), in welcher

$R^1$ für Chlor steht,

$R^2$ für Wasserstoff oder Fluor steht,

$R^3$ für Trifluormethyl, Trifluormethoxy, Trifluormethylsulfonyl oder Trifluormethylthio steht,

$R^4$ für Wasserstoff steht,

$R^5$ für Chlor steht,

$R^6$ für Nitro steht und

(A) $R^7$ für Wasserstoff, Chlor, Cyano, Hydroxy, für gegebenenfalls durch Chlor, Cyano, Carboxy, Carbamoyl, $C_1$-$C_3$-Alkoxy, $C_1$-$C_3$-Alkylthio und/oder $C_1$-$C_3$-Alkoxy-carbonyl substituiertes $C_1$-$C_2$-Alkyl, für gegebenenfalls durch Fluor, Chlor, Cyano, Methylcarbonyl oder Ethylcarbonyl substituiertes $C_1$-$C_2$-Alkoxy, für $C_3$-$C_4$-Alkenyloxy, für $C_3$-$C_4$-Alkinyloxy, für gegebenenfalls durch Fluor, Chlor, Cyano, Carboxy, Carbamoyl, $C_1$-$C_2$-Alkoxy, $C_1$-$C_2$-Alkylthio oder $C_1$-$C_2$-Alkoxy-carbonyl substituiertes $C_1$-$C_2$-Alkylthio, für jeweils gegebenenfalls durch Fluor und/oder Chlor substituiertes $C_1$-$C_2$-Alkylsulfinyl oder $C_1$-$C_2$-Alkylsulfonyl steht,

oder

(B) $R^7$ für den Rest

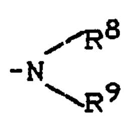

steht, worin

$R^8$ für Wasserstoff, Methyl oder Ethyl steht und

$R^9$ für Wasserstoff, Methyl, Ethyl, Methoxy, Ethoxy, Methoxycarbonyl, $C_1$-$C_2$-Alkoxycarbonyl-$C_1$-$C_2$-alkyl, Phenyl oder Benzyl steht,

oder

(C) $R^7$ für den Rest $-O-\overset{R^{10}}{\overset{|}{C}}H-\;CO-R^{11}$ oder für den Rest

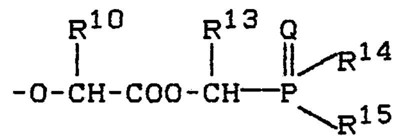

oder

für den Rest $-O-\overset{R^{10}}{\overset{|}{C}}H-\;COO-(CH_2)_m-Z$ steht, worin
$R^{10}$ für Wasserstoff oder Methyl steht,
$R^{11}$ für Chlor, Hydroxy, Methyl, Ethyl, Amino, Methylamino, Ethylamino, Dimethylamino, Diethylamino, Methylsulfonylamino, Phenylsulfonylamino oder für den Rest $-Q-R^{12}$ steht, worin
$R^{12}$ für $C_1$-$C_4$-Alkyl, $C_1$-$C_2$-Alkoxy-ethyl, $C_1$-$C_2$-Alkylthio-ethyl, Trimethylsilylmethyl, Benzyloxyethyl, $C_1$-$C_2$-Alkoxy-carbonyl-$C_1$-$C_2$-alkyl, $C_1$-$C_2$-Alkylamino-carbonyl-$C_1$-$C_2$-alkyl, Benzylthioethyl, Benzyl oder Pyrazolylmethyl steht und
Q für Sauerstoff oder Schwefel steht,
$R^{13}$ für Wasserstoff, Methyl, Phenyl oder Pyridyl steht,
$R^{14}$ für Methoxy oder Ethoxy steht,
$R^{15}$ für Methoxy oder Ethoxy steht,
Q für Sauerstoff oder Schwefel steht,
m für die Zahlen 1 oder 2 steht und
Z für Furyl, Tetrahydrofuryl, Thienyl, Perhydropyranyl, Thiazolyl oder Dioxolanyl steht,
oder

(D) $R^7$ für den Rest $-O-CH_2-\overset{R^{19}}{\overset{|}{C}}-C=N-R^{20}$ steht, worin
$R^{19}$ für Wasserstoff oder Methyl steht und
$R^{20}$ für Wasserstoff, Hydroxy, Methyl, Ethyl oder Methoxycarbonylmethyl steht.

Verwendet man für das erfindungsgemäße Verfahren (a) beispielsweise 2,6-Dichlor-4-trifluormethyl-phenol und 2-(6-Chlor-3-nitro-pyridin-2-yl-oxy)-propionsäuremethylester als Ausgangsstoffe, so kann der Verlauf des erfindungsgemäßen Verfahrens durch das folgende Formelschema wiedergegeben werden:

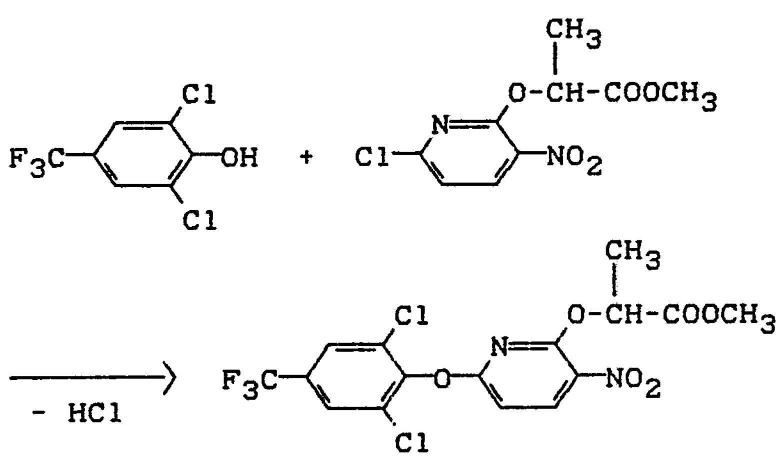


Verwendet man für das erfindungsgemäße Verfahren (b) beispielsweise 2-Amino-3-nitro-6-(2-chlor-6-fluor-4-trifluormethyl-phenoxy)-pyridin als Ausgangsstoff sowie Kaliumnitrit und Schwefelsäure als Reaktionskomponenten, so kann der Verlauf des erfindungsgemäßen Verfahrens durch das folgende Formelschema wiedergegeben werden:

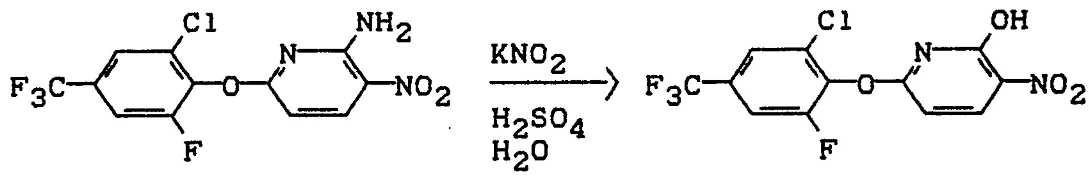


Verwendet man für das erfindungsgemäße Verfahren (c) beispielsweise 2-Methoxycarbonylmethoxy-3-nitro-6-(2,6-dichlor-4-trifluormethoxy-phenoxy)-pyridin als Ausgangsstoff und wäßrige Kalilauge als Reaktionskomponente, so kann der Reaktionsverlauf durch das folgende Formelschema wiedergegeben werden:

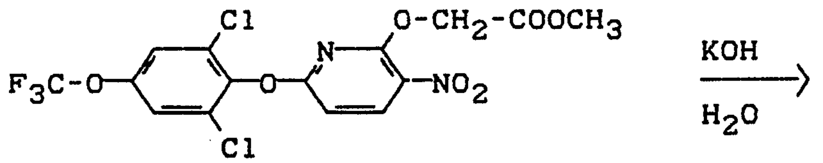


Verwendet man für das erfindungsgemäße Verfahren (d) beispielsweise 2-Carboxymethoxy-3-nitro-6-(2,6-dichlor-4-trifluormethoxy-phenoxy)-pyridin als Ausgangsstoff und Thionylchlorid als Reaktionskomponente, so kann der Reaktionsverlauf durch das folgende Formelschema wiedergegeben werden:

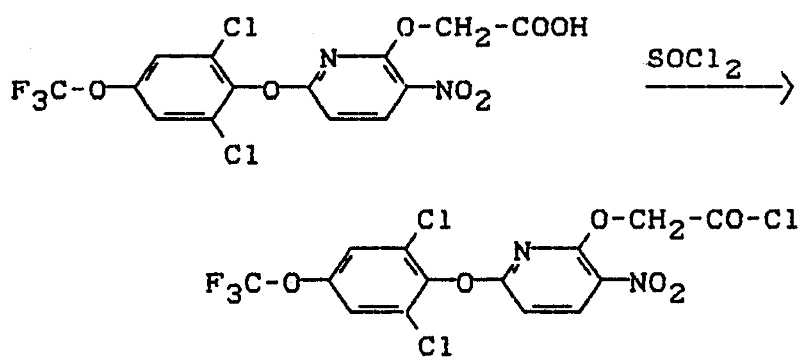


Verwendet man für das erfindungsgemäße Verfahren (e) beispielsweise 2-Chlorcarbonylmethoxy-3-cyano-6-(2,6-dichlor-4-trifluormethyl-phenoxy)-pyridin und Hydroxymethylphosphonsäure-dimethylester als Ausgangsstoffe, so kann der Reaktionsablauf durch das folgende Formelschema wiedergegeben werden:

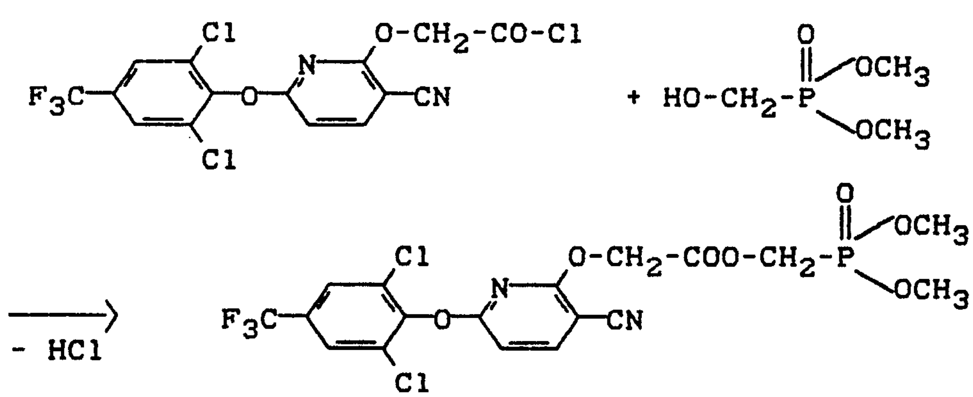


Verwendet man für das erfindungsgemäße Verfahren (f) beispielsweise 2-Amino-3-nitro-6-(2,6-dichlor-4-trifluormethyl-phenoxy)-pyridin und Methansulfonylsäurechlorid als Ausgangsstoffe, so kann der Reaktionsablauf durch das folgende Formelschema wiedergegeben werden:

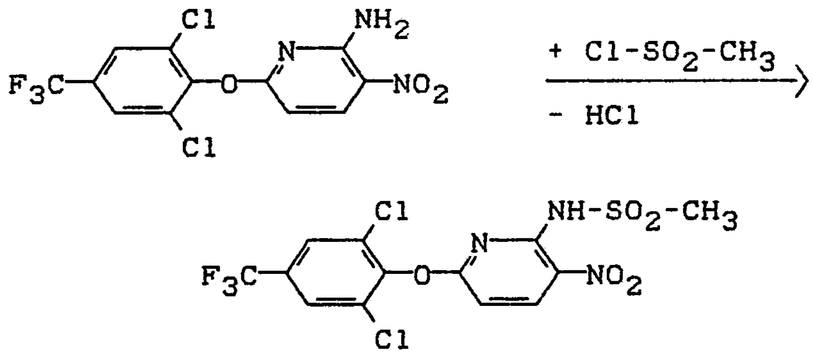


Verwendet man für das erfindungsgemäße Verfahren (g) beispielsweise 2-Methylthio-3-fluor-6-(2,6-dichlor-3-fluor-4-trifluormethyl-phenoxy)-pyridin und Hydrogenperoxid als Ausgangsstoffe, so kann der Reaktionsablauf durch das folgende Formelschema wiedergegeben werden:

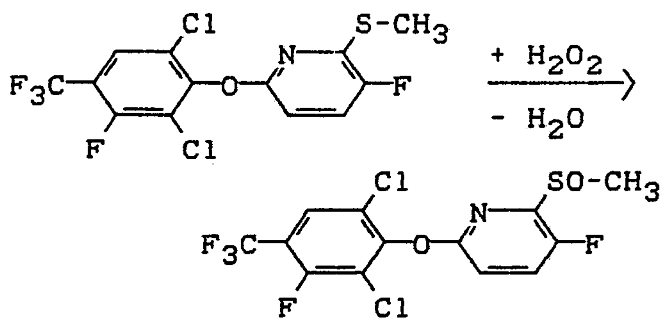


Verwendet man für das erfindungsgemäße Verfahren (h) beispielsweise 2,3-Dichlor-6-(2,6-dichlor-4-trifluormethyl-phenoxy)-pyridin und Methanol als Ausgangsstoffe, so kann der Reaktionsablauf durch das folgende Formelschema wiedergegeben werden:

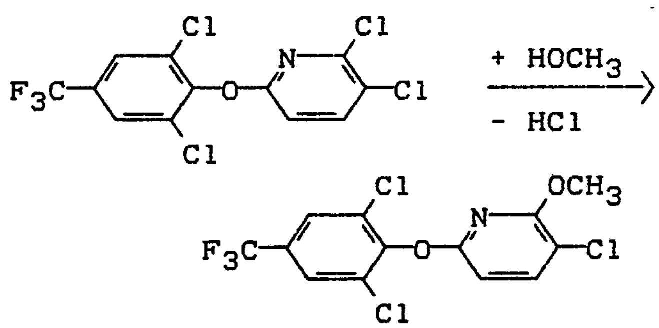


Verwendet man für das erfindungsgemäße Verfahren (i) beispielsweise 2-Amino-3-nitro-6-(2,6-dichlor-3-fluor-4-trifluormethyl-phenoxy)-pyridin und Hydrogenbromid als Ausgangsstoffe, so kann der Reaktionsablauf durch das folgende Formelschema wiedergegeben werden:

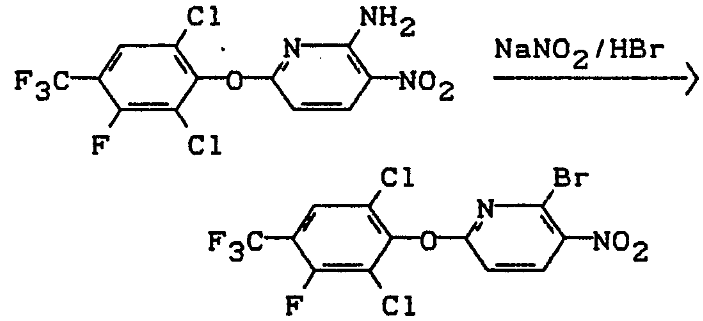


Verwendet man für das erfindungsgemäße Verfahren (j) beispielsweise 2-Carboxymethoxy-3-cyano-6-(2,6-dichlor-4-trifluormethyl-phenoxy)-pyridin und Trimethylsilylmethylchlorid als Ausgangsstoffe, so kann der Reaktionsablauf durch das folgende Formelschema wiedergegeben werden:

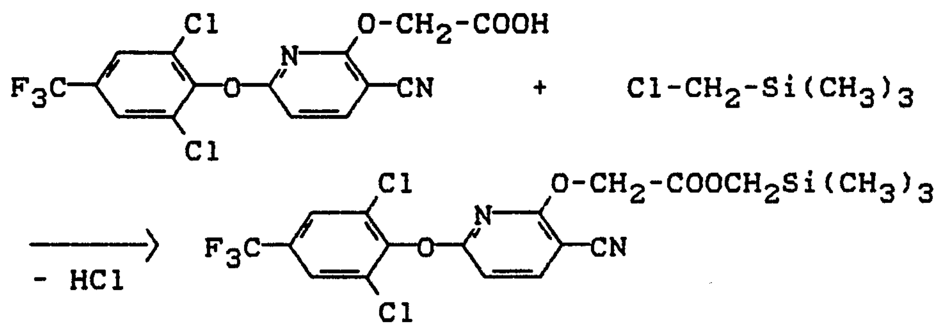


Die beim erfindungsgemäßen Verfahren (a) zur Herstellung von Verbindungen der Formel (I) als Ausgangsstoffe zu verwendenden Phenole sind durch die Formel (II) definiert.

In Formel (II) haben die Reste $R^1$, $R^2$, $R^3$, $R^4$ und $R^5$ vorzugsweise bzw. insbesondere bzw. ganz besonders bevorzugt die gleichen Bedeutungen, wie sie bereits im Zusammenhang mit der Beschreibung der erfindungsgemäßen Verbindungen der Formel (I) vorzugsweise bzw. insbesondere bzw. als ganz besonders bevorzugt angegeben wurden.

Als Beispiele für die Ausgangsstoffe der Formel (II) seien genannt:
2,6-Dichlor-4-trifluormethyl-phenol, 2,3,6-Trichlor-4-trifluormethyl-phenol, 2,6-Dichlor-3-fluor-4-trifluor methylphenol, 2-Chlor-6-fluor-4-trifluormethyl-phenol, 2,6-Dichlor-4-trifluormethoxy-phenol, 2,6-Dichlor-4-trifluormethylthio-phenol, 2,6-Dichlor-4-trifluormethylsulfinyl-phenol, 2,6-Dichlor-4-trifluormethylsulfonyl-phenol, 2,6-Dichlor-4-difluormethoxy-phenol, 2,6-Dichlor-4-chlordifluormethoxy-phenol, 2,4-Dichlor-6-trifluormethyl-phenol, 2,6-Dibrom-4-trifluormethyl-phenol, 2,3,5,6-Tetrafluor-4-trifluormethyl-phenol, 2,3,5-Trifluor-4-trifluormethyl-phenol, 3,4,5-Trifluor-2-trifluormethyl-phenol, 2-Fluor-5-chlor-4-trifluormethylphenol, 3,5-Difluor-4-trifluormethyl-phenol, 2,3-Difluor-4-trifluormethyl-phenol, 2,6-Dichlor-3,5-difluor-4-trifluormethyl-phenol, 2-Chlor-3-fluor-4-trifluormethyl-phenol, 2,5-Difluor-4-trifluormethyl-phenol und 2-Chlor-3,5,6-trifluor-4-trifluormethyl-phenol.

Die Ausgangsstoffe der Formel (II) sind bekannt und/oder können nach an sich bekannten Verfahren hergestellt werden (vgl. z. B. DE-OS 20 16 624, EP-A 63 873, US-P 4 259 532, Chem. Abstracts 60 (1964), 9170c, und das zur Herstellung der Verbindung der Formel (IIa) angegebene Verfahren).

Neu sind die Verbindungen der Formel (IIa)

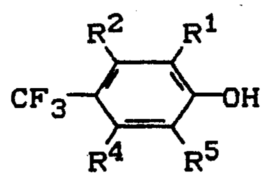
(IIa),

in welcher
$R^1$, $R^2$, $R^4$ und $R^5$ unabhängig voneinander für Wasserstoff, Fluor oder Chlor stehen,
wobei wenigstens einer dieser Substituenten für Wasserstoff steht, wenigstens ein weiterer dieser Substituenten für Fluor steht sowie wenigstens ein dritter dieser Substituenten für Fluor oder Chlor steht.

Als Beispiele für die neuen Verbindungen der Formel (II) seien genannt:

2,6-Dichlor-3-fluor-4-trifluormethyl-phenol, 2-Chlor-6-fluor-4-trifluormethyl-phenol, 2,3,5-Trifluor-4-trifluormethyl-phenol, 2-Fluor-5-chlor-trifluormethyl-phenol, 3,5-Difluor-4-trifluormethyl-phenol, 2,3-Difluor-4-trifluormethyl-phenol, 2-Chlor-3-fluor-4-trifluormethylphenol und 2,5-Difluor-4-trifluormethyl-phenol.

2,6-Dichlor-3-fluor-4-trifluormethyl-phenol wird besonders bevorzugt.

Man erhält die neuen Ausgangsstoffe der Formel (IIa), wenn man entsprechende Halogenbenzol-Derivate der Formel (IV)

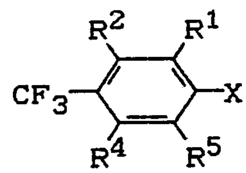

(IV)

in welcher

$R^1$, $R^2$, $R^4$ und $R^5$ die oben für die neuen Verbindungen der Formel (IIa) angegebenen Bedeutungen haben und

X für Fluor oder Chlor steht,

mit Alkalimetall-und/oder Erdalkalimetall-hydroxiden, wie z. B. Lithium-, Natrium-, Kalium-, Rubidium-, Cäsium-, Magnesium-, Calcium-oder Barium-hydroxid, vorzugsweise mit Natrium-, Kalium-und/oder Calciumhydroxid,

gegebenenfalls in Gegenwart eines Phasentransferkatalysators, wie z. B. Tetrabutylammoniumbromid, Benzyltriethylammoniumchlorid, Methyl-trioctylammoniumchlorid oder Hexadecyl-tributylphosphoniumbromid, und in Gegenwart von Verdünnungsmitteln, wie z. B. Methanol, Ethanol, Propanol, Isopropanol, Butanol, Isobutanol, sec-Butanol, tert-Butanol, Ethylenglycol, Methoxyethanol, Ethoxyethanol, Glycerin, Tetrahydrofuran, Dioxan, Pyridin, Dimethylformamid, N-Methyl-pyrrolidon, Dimethylsulfoxid, Tetramethylensulfon und/oder Wasser, vorzugsweise tert-Butanol/Wasser, tert-Butanol/Pyridin/Wasser oder tert-Butanol/Dimethylsulfoxid/Wasser bei Temperaturen zwischen 20 °C und 100 °C, vorzugsweise zwischen 30 °C und 70 °C, umsetzt und nach üblichen Methoden aufarbeitet.

Die als Zwischenprodukte zu verwendenden Halogen-benzol-Derivate sind durch die Formel (IV) allgemein definiert. In Formel (IV) stehen die Substituenten $R^1$, $R^2$, $R^4$ und $R^5$ für Wasserstoff, Fluor und/oder Chlor, wobei wenigstens einer dieser Substituenten für Wasserstoff steht und wenigstens ein weiterer dieser Substituenten für Fluor steht sowie wenigstens ein dritter dieser Substituenten für Fluor oder Chlor steht.

Als Beispiele für die Verbindungen der Formel (IV) seien genannt:

3,5-Dichlor-2,4-difluor-benzotrifluorid, 2,3,4,6-Tetrafluor-benzotrifluorid, 3-Chlor-4,5-difluor-benzotrifluorid, 2-Chlor-4,5-difluor-benzotrifluorid, 2,4,6-Trifluor-benzotrifluorid, 2,3,4-Trifluor-benzotrifluorid, 3-Chlor-2,4-difluor-benzotrifluorid und 2,4,5-Trifluorbenzotrifluorid.

Die Verbindungen der Formel (IV) sind bekannt und/oder können nach an sich bekannten Verfahren hergestellt werden (vgl. J. Chem. Soc. 1969, 211 - 217; ibid. 1971, 1547 - 1549; EP-A 34 402; US-P 4 424 396; EP-A 145 314; FR-A 2 538 380 (Chem. Abstracts 102 (1985), 61914x).

Die beim erfindungsgemäßen Verfahren (a) weiter als Ausgangsstoffe zu verwendenden Halogenpyridine sind durch die Formel (III) definiert. In Formel (III) haben die Reste $R^6$ und $R^7$ vorzugsweise bzw. insbesondere bzw. ganz besonders bevorzugt die gleichen Bedeutungen, wie sie bereits im Zusammenhang mit der Beschreibung der erfindungsgemäßen Verbindungen der Formel (I) vorzugsweise bzw. insbesondere bzw. als ganz besonders bevorzugt angegeben wurden und X steht in den bevorzugten Bereichen jeweils für Chlor.

Als Beispiele für die Ausgangsstoffe der Formel (III) seien genannt:

2-Chlor-5-nitro-pyridin, 2,6-Dichlor-5-nitro-pyridin, 2-Chlor-6-methyl-5-nitro-pyridin, 2-Chlor-6-cyano-5-nitro-pyridin, 6-Amino-2-chlor-5-nitro-pyridin, 2-Chlor-6-hydroxy-5-nitro-pyridin, 2-Chlor-6-methoxy-5-nitro-pyridin, 2-Chlor-6-ethoxy-5-nitro-pyridin, 2-Chlor-6-me thylthio-5-nitro-pyridin, 2-Chlor-6-ethylthio-5-nitropyridin, 2-Chlor-6-methylamino-5-nitro-pyridin, 2-Chlor-6-ethylamino-5-nitro-pyridin, 2-Chlor-6-dimethylamino-5-nitro-pyridin, 2-Chlor-6-methoxyamino-5-nitro-pyridin, 2-Chlor-6-ethoxyamino-5-nitro-pyridin, 2-Chlor-6-(methoxycarbonylmethylthio)-5-nitro-pyridin, 2-Chlor-6-(ethoxycarbonyl-methylthio)-5-nitro-pyridin, 2-Chlor-6-(2-methoxycarbonyl-ethylthio)-5-nitro-pyridin, 2-Chlor-6-(1-ethoxycarbonyl-ethylamino)-5-nitro-pyridin, 2-Chlor-6-methoxycarbonylmethylamino-5-nitro-pyridin, 2-Chlor-6-ethoxycarbonylmethylamino-5-nitro-pyridin,

2-Chlor-6-acetylamino-5-nitro-pyridin, (2-Chlor-5-nitro-pyridin-6-yl-oxy)-essigsäure-methylester und -ethylester, 2-(2-Chlor-5-nitro-pyridin-6-yl-oxy)-propionsäure-methylester und -ethylester.

Die Ausgangsstoffe der Formel (III) sind bekannt und/oder können nach an sich bekannten Verfahren hergestellt werden (vgl. z. B. Chemiker-Zeitung 103 (1979), 387-399; J. Heterocycl. Chem. 21 (1984), 673-679; EP-A 109 751, US-P 4 493 730 und EP-A 162 776).

Das erfindungsgemäße Verfahren (a) zur Herstellung der substituierten Phenoxypyridine der Formel (I) wird vorzugsweise unter Verwendung von Verdünnungsmitteln durchgeführt. Als Verdünnungsmittel kommen dabei vor allem polare organische Lösungsmittel, wie z. B. Aceton, Methylethylketon, Methylisopropylketon, Methylisobutylketon, Acetonitril, Propionitril, Methylenchlorid, Chloroform, Chlorbenzol, 1,2-Dichlorbenzol, 1,2-Dimethoxyethan, 1,2-Dichlorethan, Tetrahydrofuran, Dioxan, Essig säure-methylester, -ethylester, -isopropylester und -isobutylester, Dimethylformamid, Dimethylacetamid, N-Methyl-pyrrolidon, Dimethylsulfoxid und Tetramethylensulfon in Betracht.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium-und Kaliumhydroxid, Erdalkalihydroxide wie z. B. Calciumhydroxid, Alkalicarbonate und -alkoholate wie Natrium-und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 200 °C, vorzugsweise bei Temperaturen zwischen 10 °C und 150 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Umsetzungen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt.

Die Aufarbeitung erfolgt bei dem erfindungsgemäßen Verfahren (a) jeweils nach üblichen Methoden. Beispielsweise wird das Reaktionsgemisch - gegebenenfalls nach Einengen - mit Wasser verdünnt und das gewünschte Reaktionsprodukt wird mit einem mit Wasser praktisch nicht mischbaren organischen Lösungsmittel, z. B. Methylenchlorid, Chloroform, Diethylether, Toluol oder Xylol, extrahiert. Die organische Extraktionslösung wird mit Wasser gewaschen, mit einem üblichen Trockenmittel, wie z. B. Natriumsulfat, getrocknet und filtriert. Nach dem Einengen des Filtrats erhält man die Verbindungen der Formel (I) als Rohprodukte, welche auf übliche Weise, z. B. chromatographisch und/oder durch Umkristallisation gereinigt werden können.

Die beim erfindungsgemäßen Verfahren (b) als Ausgangsstoffe zu verwendenden Verbindungen sind durch die Formel (I) mit der Maßgabe, daß $R^7$ für Amino steht, allgemein definiert. In diesem Fall haben die Reste $R^1$, $R^2$, $R^3$, $R^4$, $R^5$ und $R^6$ vorzugsweise bzw. insbesondere bzw. ganz besonders bevorzugt die gleichen Bedeutungen, wie sie bereits im Zusammenhang mit der Beschreibung der erfindungsgemäßen Verbindungen der Formel (I) vorzugsweise bzw. insbesondere bzw. als ganz besonders bevorzugt angegeben werden.

Als Beispiele für die Ausgangsstoffe zu Verfahren (b) seien genannt:
2-Amino-3-nitro-6-(2,6-dichlor-4-trifluor-methyl-phenoxy)-pyridin, 2-Amino-3-nitro-6-(2,3,6-trichlor-4-trifluormethyl-phenoxy)-pyridin, 2-Amino-3-nitro-6-(2,6-dichlor-3-fluor-4-trifluormethyl-phenoxy)-pyridin, 2-Amino-3-nitro-6-(2-chlor-6-fluor-4-trifluormethyl-phenoxy)-pridin, 2-Amino-3-nitro-6-(2,3,5,6-tetrafluor-4-trifluor-methyl-phenoxy)-pyridin, 2-Amino-3-nitro-6-(2,3,5-trifluor-4-trifluormethyl-phenoxy)-pyridin, 2-Amino-3-nitro-6-(2-fluor-5-chlor-4-trifluor-methyl-phenoxy)-pyridin, 2-Amino-3-nitro-6-(3,5-difluor-4-trifluormethyl-phenoxy)-pyridin, 2-Amino-3-nitro-6-(2,3-difluor-4-trifluormethyl-phenoxy)-pyridin, 2-Amino-3-nitro-6-(2,6-dichlor-3,5-difluor-4-trifluormethyl-phenoxy)-pyridin,2-Amino-3-nitro-6-(2-chlor-3-fluor-4-trifluormethylphenoxy)-pyridin, 2-Amino-3-nitro-6-(2,5-difluor-4-trifluormethyl-phenoxy)-pyridin und 2-Amino-3-nitro-6-(2-chlor-3,5,6-trifluor-4-trifluormethyl-phenoxy)-pyridin.

Die oben beschriebenen Ausgangsstoffe der Formel (I) für Verfahren (b) sind erfindungsgemäße, neue Verbindungen; sie können nach dem erfindungsgemäßen Verfahren (a) hergestellt werden.

Verfahren (b) wird unter Verwendung von Alkalinitriten durchgeführt. Als Beispiel hierfür seien Natriumnitrit und Kaliumnitrit genannt. Vorzugsweise wird Natriumnitrit verwendet.

Verfahren (b) wird in Gegenwart einer wäßrigen Mineralsäure durchgeführt. Als Beispiele hierfür seien

Schwefelsäure, Salzsäure, Salpetersäure und Phosphorsäure - jeweils mit Wasser verdünnt - genannt. Vorzusweise wird verdünnte Schwefelsäure verwendet.

Die Reaktionstemperaturen können bei dem erfindungsgemäßen Verfahren (b) in einem größeren Bereich variiert werden. Im allgemeinen arbeitet man bei Temperaturen zwischen -10 °C und +20 °C, vorzugsweise bei Temperaturen zwischen -5 °C und +10 °C.

Das erfindungsgemäße Verfahren (b) wird im allgemeinen bei Normaldruck durchgeführt.

Zur Durchführung von Verfahren (b) werden je Mol Ausgangsverbindung der Formel (I) im allgemeinen zwischen 10 und 1000 Mol, vorzugsweise zwischen 50 und 200 Mol, Mineralsäure und zwischen 1 und 5 Mol, vorzugsweise zwischen 1,1 und 2,5 Mol, Alkalinitrit eingesetzt. Im allgemeinen wird die Ausgangsverbindung der Formel (I) in der wäßrigen Mineralsäure vorgelegt und das Alkalinitrit wird unter Kühlen in wäßriger Lösung dazu gegeben. Nach Ende der Umsetzung kann die Aufarbeitung wie oben für Verfahren (a) beschrieben durchgeführt werden.

Die beim erfindungsgemäßen Verfahren (c) als Ausgangsstoffe zu verwendenden Verbindungen sind durch die Formel (I) mit der Maßgabe, daß $R^7$ für Alkoxycarbonylalkoxy steht, allgemein definiert. In diesem Fall haben die Reste $R^1$, $R^2$, $R^3$, $R^4$, $R^5$ und $R^6$ vorzugsweise bzw. insbesondere bzw. ganz besonders bevorzugt die gleichen Bedeutungen, wie sie bereits im Zusammenhang mit der Beschreibung der erfindungsgemäßen Verbindungen der Formel (I) vorzugsweise bzw. insbesondere bzw. als ganz besonders bevorzugt angegeben wurden und $R^7$ steht vorzugsweise für $C_1$-$C_2$-Alkoxy-carbonyl-$C_1$-$C_2$-alkoxy.

Als Beispiele für die Ausgangsstoffe zu Verfahren (c) seien genannt:

2-Methoxycarbonylmethoxy-und 2-Ethoxycarbonyl-methoxy-3-nitro-6-(2,6-dichlor-4-trifluormethyl-phenoxy)-pyridin, 2-Methoxycarbonylmethoxy-und 2-Ethoxycarbonylmethoxy-3-nitro-6-(2,6-dichlor-3-fluor-4-trifluormethylphenoxy)-pyridin, 2-Methoxycarbonylmethoxy-und 2-Ethoxycarbonylmethoxy-3-nitro-6-(2,3,6-trichlor-4-trifluormethyl-phenoxy)-pyridin, 2-(1-(Methoxycarbonyl-ethoxy)-und 2-(1-Ethoxycarbonyl-ethoxy)-3-nitro-6-(2,6-dichlor-4-trifluormethyl-phenoxy)-pyridin, 2-(1-Methoxycarbonyl-ethoxy)-und 2-(1-Ethoxycarbonyl-ethoxy)-3-nitro-6-(2,6-dichlor-3-fluor-4-trifluormethyl-phenoxy)-pyridin sowie 2-(1-Methoxycarbonyl-ethoxy)- und 2-(1-Ethoxycarbonyl-ethoxy)-3-nitro-6-(2,3,6-trifluor-4-trifluormethylphenoxy)-pyridin.

Die Ausgangsstoffe der Formel (I) für Verfahren (c) sind erfindungsgemäße neue Verbindungen; sie können nach dem erfindungsgemäßen Verfahren (a) hergestellt werden.

Verfahren (c) .wird unter Verwendung von Alkalihydroxiden durchgeführt. Als Beispiele hierfür seien Lithiumhydroxid, Natriumhydroxid und Kaliumhydroxid genannt. Vorzugsweise wird Natriumhydroxid verwendet.

Verfahren (c) wird in Gegenwart von Wasser und gegebenenfalls in Gegenwart eines organischen Lösungsmittels durchgeführt. Als organische Lösungsmittel werden vorzugsweise Alkohole, wie z. B. Methanol oder Ethanol eingesetzt.

Zum Ansäuern werden bei Verfahren (c) die üblichen Mineralsäuren, wie z. B. Salzsäure oder Schwefelsäure, verwendet.

Die Reaktionstemperaturen können bei dem erfindungsgemäßen Verfahren (c) in einem größeren Bereich variiert werden. Im allgemeinen arbeitet man bei Temperaturen zwischen 10 °C und 150 °C, vorzugsweise bei Temperaturen zwischen 20 °C und 100 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung von Verfahren (c) werden je Mol Aus gangsverbindung der Formel (I) im allgemeinen zwischen 0,1 und 10 Mol, vorzugsweise zwischen 0,5 und 2 Mol, Alkalihydroxid eingesetzt. Die Reaktionskomponenten werden im allgemeinen bei Raumtemperatur zusammengegeben und das Reaktionsgemisch wird, gegebenenfalls bei erhöhter Temperatur, bis zum Reaktionsende gerührt. Das - gegebenenflls nach Einengen, Abkühlen und Ansäuern - kristallin anfallende Reaktionsprodukt kann durch Absaugen isoliert werden.

Die beim erfindungsgemäßen Verfahren (d) als Ausgangsstoffe zu verwendenden Verbindungen sind durch die Formel (I) mit der Maßgbe, daß $R^7$ für Carboxyalkoxy steht, allgemein definiert. In diesem Fall haben die Reste $R^1$, $R^2$, $R^3$, $R^4$, $R^5$ und $R^6$ vorzugsweise bzw. insbesondere bzw. ganz besonders bevorzugt die gleichen Bedeutungen, wie sie bereits im Zusammenhang mit der Beschreibung der erfindungsgemäßen Verbindungen der Formel (I) vorzugsweise bzw. Insbesondere bzw. als ganz besonders bevorzugt angegeben wurden und $R^7$ steht vorzugsweise für Carboxy-$C_1$-$C_2$-alkoxy.

Als Beispiele für die Ausgangsstoffe zu Verfahren (d) seien genannt:

2-Carboxymethoxy-und 2-(1-Carboxy-ethoxy)-3-nitro-6-(2,6-dichlor-4-trifluormethyl-phenoxy)-pyridin, 2-Carboxymethoxy und 2-(1-Carboxy-ethoxy)-3-nitro-6-(2,6-dichlor-3-fluor-4-trifluormethyl)-phenoxy)-pyridin sowie 2-Carboxymethoxy-und 2-(1-Carboxy-ethoxy)-3-nitro-6-(2,3,6-trichlor-4-trifluormethyl-phenoxy)-pyridin.

Die Ausgangsstoffe der Formel (I) für Verfahren (d) sind erfindungsgemäße neue Verbindungen; sie

können nach dem erfindungsgemäßen Verfahren (c) hergestellt werden.

Verfahren (d) wird unter Verwendung eines Halogenierungsmittels durchgeführt. Es können die üblichen Mittel für die Umsetzung von Carbonsäuren zu Carbonsäurehalogeniden eingesetzt werden. Als Beispiele hierfür seien Phosgen, Thionylchlorid, Phosphorylchlorid und Benzotrichlorid genannt. Vorzugsweise Wird Thionylchlorid als Halogenierungsmittel verwendet.

Verfahren (d) wird gegebenenfalls in Gegenwart eines Verdünnungsmittels verwendet. Vorzugsweise kommen inerte organische Lösungsmittel aus der Reihe der halogenierten Kohlenwasserstoffe, wie z. B. Methylenchlorid, Chloroform, Tetrachlormethan oder 1,2-Dichlorethan, in Betracht.

Die Reaktionstemperaturen können bei dem erfindungsgemäßen Verfahren (d) in einem größeren Bereich variiert werden. Im allgemeinen arbeitet man bei Temperaturen zwischen 0 °C und 100 °C, vorzugsweise bei Temperaturen zwischen 10 °C und 90 °C.

Verfahren (d) wird im allgemeinen bei Normaldruck durchgeführt.

Zur Durchführung von Verfahren (d) werden je Mol Ausgangsverbindung der Formel (I) im allgemeinen zwischen 1 und 100 Mol, vorzugsweise zwischen 2 und 50 Mol, Halogenierungsmittel eingesetzt. Die Reaktionskomponenten werden im allgemeinen bei Raumtemperatur zusammengegeben und das Reaktionsgemisch wird, gegebenenfalls bei erhöhter Temperatur bis zum Ende der Umsetzung gerührt. Das nach Abdestillieren der flüchtigen Komponenten unter vermindertem Druck verbleibende Reaktionsprodukt kann durch Umkristallisation gereinigt werden, aber auch ohne weitere Reinigung für Folgeumsetzungen eingesetzt werden.

Die beim erfindungsgemäßen Verfahren (e) als Ausgangsstoffe zu verwendenden Verbindungen sind durch die Formel (I) mit der Maßgabe, daß $R^7$ für Chlorcarbonylalkoxy (-O-CH($R^{10}$)-CO-Cl) steht, allgemein definiert. In diesem Fall haben die Reste $R^1$, $R^2$, $R^3$, $R^4$, $R^5$ und $R^6$ vorzugsweise bzw. insbesondere bzw. ganz besonders bevorzugt die gleichen Bedeutungen, wie sie bereits im Zusammenhang mit der Beschreibung der erfindungsgemäßen Verbindungen der Formel (I) vorzugsweise bzw. insbesondere bzw. als ganz besonders bevorzugt angegeben wurden und $R^7$ steht vorzugsweise für Chlorcarbonyl-$C_1$-$C_2$-alkoxy.

Als Beispiele für die Ausgangsstoffe zu Verfahren (e) seien genannt:

2-Chlorcarbonylmethoxy-und 2-(1-Chlorcarbonyl-ethoxy)-3-nitro-6-(2,6-dichlor-4-trifluormethyl-phenoxy)-pyridin, 2-Chlorcarbonylmethoxy-und 2-(1-Chlorcarbonyl-ethoxy-3-nitro-6-(2,6-dichlor-3-fluor-4-trifluormethyl-phenoxy)-pyridin sowie 2-Chlorcarbonylmethoxy-und 2-(1-Chlor-carbonyl-ethoxy)-3-nitro-6-(2,3,6-trichlor-4-trifluormethyl-phenoxy)-pyridin.

Die Ausgangsstoffe der Formel (I) für Verfahren (e) sind erfindungsgemäße Verbindungen; sie können nach dem erfindungsgemäßen Verfahren (d) hergestellt werden.

Bei den als weitere Ausgangsstoffe für Verfahren (e) einzusetzenden Verbindungen haben m, Q, $R^{11}$, $R^{13}$, $R^{14}$, $R^{15}$, $R^{16}$, $R^{17}$ und Z vorzugsweise bzw. insbesondere bzw. ganz besonders bevorzugt die gleichen Bedeutungen, wie sie bereits im Zusammenhang mit der Beschreibung der erfindungsgemäßen Verbindungen der Formel (I) vorzugsweise bzw. insbesondere bzw. als ganz besonders bevorzugt angegeben wurden.

Als Beispiele für diese Ausgangsstoffe zu Verfahren (e) seien genannt:

Methylamin, Ethylamin, Propylamin, Isopropylamin, Cyanamid, Dimethylamin, Diethylamin, Hydroxylamin, O-Methylhydroxylamin, Hydrazin, Methylsulfonylhydrazin, Methanol, Ethanol, Propanol, Isopropanol, Butanol, Isobutanol, sec-Butanol, 2-Methoxy-ethanol, 2-Ethoxy-ethanol, 2-Methylthio-ethanol, 2-Ethylthio-ethanol, 2-Benzyloxyethanol, 2-Benzylthio-ethanol, Hydroxymethanphosphonsäure-diethylester und -dimethylester, 1-Hydroxy-ethanphosphonsäure-dimethylester und -diethylester, 1-Hydroxy -1-phenyl-methanphosphonsäure-dimethylester und -diethylester, Acetonoxim, 3-Hydroxyfuran, Furfurylalkohol, Perhydrofurfurylalkohol, Milchsäure-methylester und -ethylester und Glycolsäure-methylester und -ethylester.

Diese Verbindungen sind bekannte Synthesechemikalien.

Das erfindungsgemäße Verfahren (e) wird vorzugsweise un ter Verwendung eines Säureakzeptors durchgeführt. Insbesondere kommen diejenigen Säurebindemittel in Betracht, die bereits oben im Zusammenhang mit der Beschreibung des erfindungsgemäßen Verfahrens (a) genannt wurden.

Das erfindungsgemäße Verfahren (e) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (e) in einem größeren Bereich variiert werden. Im allgemeinen arbeitet man bei Temperaturen zwischen 0 °C und 100 °C, vorzugsweise bei Temperaturen zwischen 10 °C und 50 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung von Verfahren (e) werden die Ausgangsstoffe im allgemeinen in angenähert äquimolaren Mengen eingesetzt. Die Umsetzung und die Aufarbeitung können wie oben für das erfindungsgemäße Verfahren (a) beschrieben durchgeführt werden.

Die beim erfindungsgemäßen Verfahren (f) als Ausgangsstoffe zu verwendenden Verbindungen sind durch die Formel (I) mit der Maßgabe, daß $R^7$ für den Rest -NH-$R^8$ steht, allgemein definiert. In diesem Fall haben die Reste $R^1$, $R^2$, $R^3$, $R^4$, $R^5$, $R^6$ und $R^8$ vorzugsweise bzw. insbesondere bzw. ganz besonders bevorzugt die gleichen Bedeutungen, wie sie bereits im Zusammenhang mit der Beschreibung der erfindungsgemäßen Verbindungen der Formel (I) vorzugsweise bzw. insbesondere bzw. als ganz besonders bevorzugt angegeben wurden.

Beispiele für diese Ausgangsstoffe wurden bereits bei der Beschreibung der Ausgangsstoffe für das erfindungsgemäße Verfahren (b) einzeln genannt.

Die Ausgangsstoffe der Formel (I) für Verfahren (f) sind erfindungsgemäße Verbindungen; sie können nach dem erfindungsgemäßen Verfahren (a) hergestellt werden.

Verfahren (f) wird unter Verwendung von Alkoxycarbonyl-, Alkylcarbonyl-, Arylcarbonyl-, Aryloxycarbonyl-, Alkyl sulfonyl-oder Arylsulfonyl-halogeniden bzw. entsprechenden Anhydriden bzw. von Alkyl-oder Arylisocyanaten durchgeführt.

Als Beispeiele hierfür seien genannt:

Chlorameisensäure-methylester, -ethylester und -propylester, Acetylchlorid, Acetanhydrid, Propionsäure-, Buttersäure-, Isobuttersäure-, Valeriansäure-und Isovaleriansäure-chlorid, Benzoylchlorid, Chlorameisensäurephenylester, Methansulfonsäurechlorid, Benzolsulfonsäurechlorid, Methyl-, Ethyl-, Propyl-, und Butyl-isocyanat sowie Phenylisocyanat.

Diese Ausgangsstoffe sind bekannte Synthesechemikalien.

Verfahren (f) wird gegebenenfalls in Gegenwart eines Säureakzeptors durchgeführt. Vorzugsweise kommen diejenigen Säurebindemittel in Betracht, die bereits oben im Zusammenhang mit der Beschreibung des erfindungsgemäßen Verfahrens (a) genannt wurden.

Verfahren (f) wird vorzugsweise unter Verwendung von Verdünnungsmitteln duchgeführt. Insbesondere kommen diejeningen Verdünnungsmittel in Betracht, die bereits oben bei der Beschreibung des erfindungsgemäßen Verfahrens (e) genannt wurden. Im Falle der Umsetzung mit Säureanhydriden können auch die entsprechenden Säuren als Verdünnungsmittel verwendet werden.

Die Reaktionstemperaturen können bei dem erfindungsgemäßen Verfahren (f) in einem größeren Bereich variiert werden. Im allgemeinen arbeitet man bei Temperaturen zwischen 0 °C und 100 °C, vorzugsweise bei Temperaturen zwischen 10 °C und 80 °C.

Das erfindungsgemäße Verfahren (f) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (f) werden die jeweils benötigten Ausgangstoffe im allgemeinen in angenähert äquimolaren Mengen eingesetzt. Es ist jedoch auch möglich, ein der beiden jeweils eingesetzten Komponenten in einem größeren Überschuß zu verwenden.

Die Umsetzung und die Aufarbeitung können wie oben für das erfindungsgemäße Verfahren (a) beschrieben durchgeführt werden.

Die beim erfindungsgemäßen Verfahren (g) als Ausgangsstoffe zu verwendenden Verbindungen sind durch die Formel (I) mit der Maßgabe, daß $R^7$ für Alkylthio steht, allgemein definiert. In diesem Fall haben die Reste $R^1$, $R^2$, $R^3$, $R^4$, $R^5$ und $R^6$ vorzugsweise bzw. insbesondere bzw. ganz besonders bevorzugt die gleichen Bedeutungen, wie sie bereits im Zusammenhang mit der Beschreibung der erfindungsgemäßen Vebindungen der Formel (I) vorzugsweise bzw. insbesondere bzw. als ganz besonders bevorzugt angegeben wurden und $R^7$ steht vorzugsweise für $C_1$-$C_3$-Alkylthio.

Als Beispiele für die Ausgangsstoffe zu Verfahren (g) seien genannt:

2-Methylthio-, 2-Ethylthio-und 2-Propylthio-3-nitro-6-(2,6-dichlor-4-trifluormethyl-phenoxy)-pyridin, 2-Methylthio-, 2-Ethylthio-und 2-Propylthio-3-nitro-6-(2,6-dichlor-3-fluor-4-trifluormethyl-phenoxy)-pyridin sowie 2-Methylthio-, 2-Ethylthio-und 2-Propylthio-3-nitro-6-(2,3,6-trichlor-4-trifluormethyl-phenoxy)-pyridin.

Die Ausgangsstoffe der Formel (I) für Verfahren (g) sind erfindungsgemäße Verbindungen; sie können nach dem erfindungsgemäßen Verfahren (a) hergestellt werden.

Das erfindungsgemäße Verfahren (g) wird unter Verwendung eines Oxidationsmittels durchgeführt. Es können die üblichen Mittel zur Oxidation von Thioethern zu Sulfoxiden bzw. zu Sulfonen verwendet werden

(vgl. Tetrahedron 42 (1986), 5459 - 5495; Phosphorus and Sulfur 22 (1985), 5 - 11).
Als Beispiele für geeignete Oxidationsmittel seien genannt: Hydrogenperoxid ($H_2O_2$) oder daraus mit Carbonsäuren, wie z. B. Ameisensäure, Essigsäure, Propionsäure oder Benzoesäure, erzeugte Persäuren sowie Chlor und hypochlorige Säure oder deren Salze.

Verfahren (g) wird gegebenenfalls in Gegenwart eines Katalysators durchgeführt. Es können die üblichen Phasentransfer-Katalysatoren, wie z. B. Tetrabutylammoniumbromid, Benzyl-triethylammoniumchlorid und Methyltrioctylammoniumchlorid und/oder die für Oxidationsreak tionen üblichen Katalysatoren; wie z. B. Ammoniummolybdat, Natriumwolframat, Natriumvanadinat, Selendioxid oder Titan(III)-chlorid, verwendet werden.

Verfahren (g) wird vorzugsweise unter Verwendung von Verdünnungsmitteln durchgeführt. Insbesondere kommen diejenigen Verdünnungsmittel in Betracht, die bereits oben bei der Beschreibung des erfindungsgemäßen Verfahrens (e) genannt wurden, ferner jedoch auch Wasser und Alkohole, wie z. B. Methanol, Ethanol und Isopropanol.

Die Reaktionstemperaturen können bei dem erfindungsgemäßen Verfahren (g) in einem größeren Bereich variiert werden. Im allgemeinen arbeitet man bei Temperaturen zwischen 0 °C und 80 °C, vorzugsweise bei Temperaturen zwischen 0 °C und 50 °C.

Verfahren (g) wird im allgemeinen bei Normaldruck durchgeführt.

Zur Durchführung von Verfahren (g) werden je Mol Ausgangsverbindung der Formel (I) im allgemeinen zwischen 1 und 10 Mol, vorzugsweise zwischen 1,1 und 3,0 Mol (bzw. Moläquivalente) des Oxidationsmittels eingesetzt. Im allgemeinen wird die Ausgangsverbindung der Formel (I) vorgelegt und das Oxidationsmittel eindosiert; dann wird das Reaktionsgemisch bis zum Ende der Umsetzung gerührt. Die Aufarbeitung kann wie oben für Verfahren (a) beschrieben durchgeführt werden.

Die beim erfindungsgemäßen Verfahren (h) als Ausgangs stoffe zu verwendenden Verbindungen sind durch die Formel (I) mit der Maßgabe, daß $R^7$ für Halogen steht, allgemein definiert. In diesem Fall haben die Rest $R^1$, $R^2$, $R^3$, $R^4$, $R^5$ und $R^6$ vorzugsweise bzw. insbesondere bzw. ganz besonders bevorzugt die gleichen Bedeutungen, wie sie bereits im Zusammenhang mit der Beschreibung der erfindungsgemäßen Vebindungen der Formel (I) vorzugsweise bzw. insbesondere bzw. als ganz besonders bevorzugt angegeben wurden und $R^7$ steht vorzugsweise für Chlor.

Als Beispiele für die Ausgangsstoffe zu Verfahren (h) seien genannt:
2-Chlor-3-nitro-6-(2,6-dichlor-4-trifluormethyl-phenoxy)-pyridin, 2-Chlor-3-nitro-6-(2,6-dichlor-3-fluor-4-trifluormethyl-phenoxy)-pyridin und 2-Chlor-3-nitro-6-(2,3,6-trichlor-4-trifluormethyl-phenoxy)-pyridin.

Die Ausgangsstoffe der Formel (I) für Verfahren (h) sind erfindungsgemäße Verbindungen; sie können nach dem erfindungsgemäßen Verfahren (a) hergestellt werden.

Das erfindungsgemäße Verfahren (h) wird unter Verwendung von Alkanolen und/oder Alkalimetallsalzen von Alkanolen durchgeführt. Vorzugsweise werden $C_1$-$C_4$-Alkanole und deren Natrium-oder Kalium-salze eingesetzt. Als Beispiele seien Methanol, Ethanol, Propanol, Isopropanol, Butanol, Isobutanol und sec-Butanol sowie deren Natrium-und Kaliumsalze genannt.

Die Reaktionstemperaturen können bei dem erfindungsgemäßen Verfahren (h) in einem größeren Bereich variiert werden. Im allgemeinen arbeitet man bei Temperaturen zwischen 0 °C und 80 °C, vorzugsweise bei Temperaturen zwischen 10 °C und 50 °C.

Verfahren (h) wird im allgemeinen bei Normaldruck durchgeführt.

Zur Durchführung von Verfahren (h) setzt man im allgemeinen je Mol Ausgangsverbindung der Formel (I) zwischen 1 und 3 Mol, vorzugsweise zwischen 1,1 und 1,5 Mol, Alkalimetallsalz eines Alkanols und/oder zwischen 1 und 100 Mol, vorzugsweise zwischen 10 und 50 Mol, eines Alkanols ein. Die Raktionskomponenten werden im allgemeinen bei Raumtemperatur zusammengegeben und das Reaktionsgemisch wird bis zum Ende der Umsetzung gerührt. Die Aufarbeitung kann wie oben für Verfahren (a) beschrieben durchgeführt werden.

Die beim erfindungsgemäßen Verfahren (i) als Ausgangsstoffe zu verwendenden Verbindungen sind durch die Formel (I) mit der Maßgabe, daß $R^7$ für Amino steht, allgemein definiert. In diesem Fall haben die Reste $R^1$, $R^2$, $R^3$, $R^4$, $R^5$ und $R^6$ vorzugsweise bzw. insbesondere bzw. ganz besonders bevorzugt die gleichen Bedeutungen, wie sie bereits im Zusammenhang mit der Beschreibung der erfindungsgemäßen Vebindungen der Formel (I) vorzugsweise bzw. insbesondere bzw. als ganz besonders bevorzugt angegeben wurden. Beispiele für diese Ausgangsstoffe wurden bereits bei der Beschreibung der Ausgangsstoffe für das erfindungsgemäße Vefahren (b) einzeln genannt.

Die Ausgangsstoffe der Formel (I) für Verfahren (i) sind erfindungsgemäße Verbindungen; sie können nach dem erfindungsgemäßen Verfahren (a) hergestellt werden.

Verfahren (i) wird unter Verwendung von Alkalimetallnitriten durchgeführt. Als Beispiele hierfür seien Natriumnitrit und Kaliumnitrit genannt. Vorzugsweise wird Natiumnitrit verwendet.

Verfahren (i) wird unter Verwendung eines Hydrogen-oder Metall-halogenids bzw. eines Metallhalogenid-Komplexes durchgeführt. Als Beispiele hierfür seien genannt:

Hydrogen-fluorid, - chlorid, -bromid und -iodid, Lithium-, Natrium-und Kalium-fluorid, Lithium-, Natrium-und Kalium-chlorid, Lithium-, Natrium-und Kalium-bromid, Lithium-, Natrium und Kalium-iodid sowie Lithium-, Natrium-und Kalium-tetrafluoroborat.

Säuren, welche beim erfindungsgemäßen Verfahren (i) eingesetzt werden können, sind die oben genannten Hydrogenhalogenide, wie z. B. Hydrogen-fluorid, -chlorid, -bromid und -iodid, daneben jedoch auch organische Säuren, wie z. B. Ameisensäure, Essigsäure, Propionsäure oder Methansulfonsäure.

Verfahren (i) wird gegebenenfalls in Gegenwart eines Katalysators durchgeführt. Es kommen vorzugsweise Kupfer und Kupferverbindungen, wie z. B. Kupferchlorid-, -bromid, -iodid, -nitrat und -sulfat in Betracht.

Verfahren (i) wird in Gegenwart eines Verdünnungsmittels durchgeführt. Als Verdünnungsmittel kommen neben Wasser vor allem organische Säure, wie z. B. Ameisensäure, Essigsäure, Propionsäure und Methansulfonsäure in Betracht.

Die Reaktionstemperaturen können bei dem erfindungsgemäßen Verfahren (i) in einem größeren Bereich variiert werden. Im allgemeinen arbeitet man bei Temperaturen zwischen 0 °C und 80 °C, vorzugsweise bei Temperaturen zwischen 0 °C und 50 °C.

Verfahren (i) wird im allgemeinen bei Normaldruck durchgeführt.

Zur Durchführung von Verfahren (i) setzt man je Mol Ausgangsverbindung der Formel (I) zwischen 1 und 3 Mol, vorzugsweise zwischen 1,1 und 1,5 Mol, Alkalimetallnitrit, zwischen 1 und 100 Mol, vorzugsweise zwischen 2 und 50 Mol eines Hydrogen-oder Metall-halogenids bzw. Metallhalogenid-Komplexes und die angenähert äquimolare Menge einer Säure ein. Die Reaktionskomponenten werden im allgemeinen bei Raumtemperatur oder unter Kühlung zusammengegeben und das Reaktionsgemisch wird bis zum Ende der Umsetzung gerührt. Die Aufarbeitung kann wie oben für Verfahren (a) beschrieben durchgeführt werden.

Die beim erfindungsgemäßen Verfahren (j) als Ausgangsstoffe zu verwendenden Verbindungen sind durch die Formel (I) mit der Maßgabe, daß $R^7$ für den Rest -O-CH($R^{10}$)-COOH steht, allgemein definiert. In diesem Fall haben die Reste $R^1$, $R^2$, $R^3$, $R^4$, $R^5$, $R^6$ und $R^{10}$ vorzugsweise bzw. insbesondere bzw. ganz besonders bevorzugt die gleichen Bedeutungen, wie sie bereits im Zusammenhang mit der Beschreibung der erfindungsgemäßen Vebindungen der Formel (I) vorzugsweise bzw. insbesondere bzw. als ganz besonders bevorzugt angegeben wurden.

Als Beispiele für die Ausgangsstoffe der Formel (I) zu Verfahren (j) seien genannt:

2-Carboxymethoxy-und 2-(1-Carboxy-ethoxy)-3-nitro-6-(2,6-dichlor-4-trifluor-methyl-phenoxy)-pyridin, 2-Carboxymethoxy-und 2-(1-Carboxy-ethoxy)-3-nitro-6-(2,6-dichlor-3-fluor-6-(2,6-dichlor-3-fluor-4-trifluormethylphenoxy)-pyridin sowie 2-Carboxymethoxy-und 2-(1-Carboxy-ethoxy-3-nitro-6-(2,3,6-trichlor-4-trifluormethylphenoxy)-pyridin.

Die Ausgangsstoffe der Formel (I) für Verfahren (j) sind erfindungsgemäße Verbindungen; sie können nach dem erfindungsgemäßen Verfahren (c) hergestellt werden.

Das erfindungsgemäße Verfahren (j) wird unter Verwendung von Trialkylsilylalkylhalogeniden durchgeführt. Vorzugsweise werden Trimethylsilyl-$C_1$-$C_4$-alkylchloride oder -bromide, insbesondere Trimethylsilylmethylchlorid, eingesetzt.

Die bei Verfahren (j) zu verwendenden Trialkylsilylalkylhalogenide sind bekannte Synthesechemikalien.

Verfahren (j) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden. Aceton, Acetonitril und Dimethylformamid werden besonders bevorzugt.

Verfahren (j) wird vorzugsweise in Gegenwart eines Säureakzeptors durchgeführt. Es kommen vor allem diejenigen Säureakzeptoren in Betracht, die bereits bei der Beschreibung des erfindungsgemäßen Verfahrens (a) genannt wurden. 1,8-Diazabicyclo-[5.4.0]-undec-7-en (DBU) wird besonders bevorzugt.

Die Reaktionstemperaturen können bei dem erfindungsgemäßen Verfahren (j) in einem größeren Bereich variiert werden. Im allgemeinen arbeitet man bei Temperaturen zwischen 0 °C und 100 °C, vorzugsweise bei Temperaturen zwischen 20 °C und 80 °C.

Das erfindungsgemäße Verfahren (j) wird im allgemeinen unter Normaldruck durchgeführt. Es ist jedoch auch möglich, unter erhöhtem oder vermindertem Druck zu arbeiten.

Zur Durchführung von Verfahren (j) setzt man je Mol Ausgangsverbindung der Formel (I) im allgemeinen zwischen 1 und 3 Mol, vorzugsweise zwischen 1,1 und 2,5 Mol, Trialkylsilylalkylhalogenid ein.

Umsetzung und Aufarbeitung können wie oben für Verfahren (a) beschrieben durchgeführt werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle

Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Wirkstoffe eignen sich sehr gut zur Bekämpfung monokotyler und dikotyler Unkräuter im Vorauflauf-und im Nachauflauf-Verfahren.

Zur selektiven Bekämpfung von dikotylen Unkräutern in monokotylen Kulturen im Nachauflauf-Verfahren sind die erfindungsgemäßen Wirkstoffe ganz besonders gut geeignet.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur-und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymer verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage, ferner 2,4-Dichlorphenoxyessigsäure; 2,4-Dichlorphenoxypropionsäure; 4-(2,4-Dichlorphenoxy)-buttersäure; (2-Methyl-4-chlorphenoxy)-essigsäure; (4-Chlor-2-methylphenoxy)-propionsäure; 3,5,6-Trichlor-2-pyridyloxyessigsäure; 2-[4-(2,4-Dichlorphenoxy)-phenoxyl]-propionsäure, deren Methyl-oder deren Ethylester; 2-{4-[(6-Chlor-2-benzoxazolyl)-oxy]-phenoxy}-propansäure, deren Methyl-oder deren Ethylester; 2-[4-(3,5-Dichlorpyrid-2-yloxy)-phenoxy]-propionsäure-(trimethylsilylmethylester); 3,6-Dichlor-2-pyridincarbonsäure; N-(1-Ethylpropyl)-3,4-dimethyl-2,6-dinitroanilin; Methyl-5-(2,4-dichlorphenoxy)-2-nitrobenzoat; N,N-Dimethyl-N'-(3-chlor-4-methylphenyl)-harnstoff; N,N-Dimethyl-N'-(4-isopropylphenyl)-harnstoff; Methyl-6,6-dimethyl-2,4-dioxo-3-[1-(2-propenyloxyamino)-butyliden]-cyclohexancarbonsäure; Methyl-2-[4,5-dihydro-4-methyl-4-(1-methylethyl)-5-oxo-1H-imidazol-2-yl]-4(5)-methylbenzoat; 3,5-Dibrom-4-hydroxy-benzonitril; 3,5-Diiod-4-hydroxybenzonitril; N-Methyl-2-(1,3-benzthiazol-2-yloxy)-acetanilid; 2-Chlor-N-{[(4-methoxy-6-methyl-1,3,5-triazin-2-yl)-amino]-carbonyl}-benzolsulfonamid; 2-{[[((4-Methoxy-6-methyl-1,3,5-triazin-2-yl)-amino)-carbonyl]-amino]-sulfonyl}-benzoesäure oder deren Methylester; 3-[[[[(4-Methoxy-6-methyl-1,3,5-triazin-2-yl)-amino]-carbonyl]-amino]-sulfonyl]-thiophen-2-carbonsäure-methylester; 4-Ethylamino-2-t-butylamino-6-me thylthio-s-triazin; 3-Isopropyl-2,1,3-benzothiadiazin-4-on-2,2-dioxid und 0-(6-Chlor-3-phenyl-pyridazin-4-yl)-S-octyl-thiocarbonat. Einige Mischungen zeigen auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen,

Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe gehen aus den nachfolgenden Beispielen hervor.

Herstellungsbeispiele

Beispiel 1

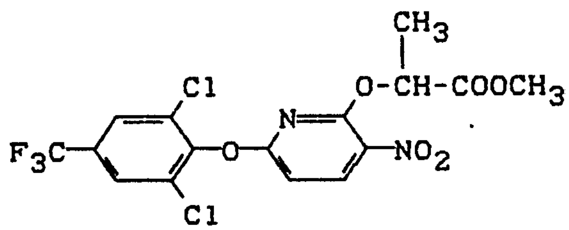

Verfahren (a)

7,8 g (0,03 Mol) 2-(3-Nitro-6-chlor-pyridinyl-2-oxy)-propionsäuremethylester, 6,9 g (0,03 Mol) 2,6-Dichlor-4-trifluormethyl-phenol und 8,3 g (0,06 Mol) Kaliumcarbonat werden in 60 ml Dimethylsulfoxid 24 Stunden bei 100 °C gerührt. Der Ansatz wird mit 500 ml Wasser verdünnt und zweimal mit je 150 ml Methylenchlorid extrahiert. Die vereinigten Methylenchloridphasen werden mit Wasser gewaschen, über Natriumsulfat getrocknet, filtriert und eingeengt. Das zurückbleibende Oel wird mit einem Gemisch von 4 Teilen n-Hexan und einem Teil Essigester als Laufmittel über eine Kieselgelsäule chromatographiert.

Man erhält 2,9 g (21% der Theorie) 2-[3-Nitro-6-(2,6-dichlor-4-trifluormethylphenoxy]-pyridinyl-2-oxy]-propionsäure-methylester als farblose Kristalle vom Schmelzpunkt 108 - 109 °C.

Beispiel 2

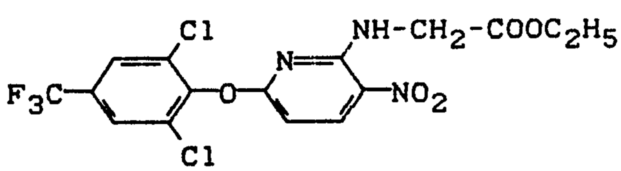

Verfahren (a)

3,2 g (0,0125 Mol) 2,6-Dichlor-4-trifluormethyl-phenol, 3,2 g (0,0125 Mol) 2-Ethoxycarbonylmethylamino-3-nitro-6-chlor-pyridin und 1,4 g (0,0125 Mol) 1,4-Diazabicyclo-[2,2,2]-octan werden in 100 ml Chloroform 9 Stunden bei 60 °C gerührt; die Chloroformphase wird zweimal mit Wasser gewaschen, über Natriumsulfat getrocknet, filtriert und eingeengt. Das verbleibende Öl wird mit Ligroin kristallisiert, abgesaugt und getrocknet.

Man erhält 3,6 g (63 % der Theorie) 2-Ethoxy-carbonylmethylamino-3-nitro-6-(2,6-dichlor-4-trifluormethylphenoxy)-pyridin vom Schmelzpunkt 140 - 145 °C.

Beispiel 3

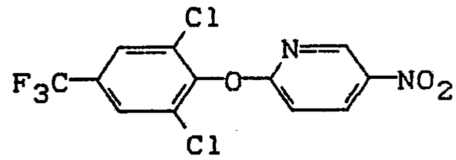

Verfahren (a)

17,4 g (0,075 Mol) 2,6-Dichlor-4-trifluormethylphenol, 8 g (0,050 Mol) 2-Chlor-5-nitro-pyridin und 10,4 g (0,075 Mol) Kaliumcarbonat werden in 200 ml Dimethylsulfoxid 48 Stunden bei 90°C gerührt. Nach dem Abkühlen auf 20 - 30 °C wird der Ansatz auf Eis gegossen und zweimal mit jeweils 250 ml Chloroform extrahiert. Die vereinigten Chloroformphasen werden mit Wasser gewaschen, über Natriumsulfat getrocknet, filtriert und eingeengt. Der Rückstand wird mit Methylenchlorid als Laufmittel über eine Kieselgelsäule chromatographiert.

Nach dem Eindampfen des Eluats erhält man 2,4 g (13% der Theorie) 3-Nitro-6-(2,6-dichlor-4-trifluormethyl-phenoxy)-pyridin vom Schmelzpunkt 102 - 108 °C.

Beispiel 4

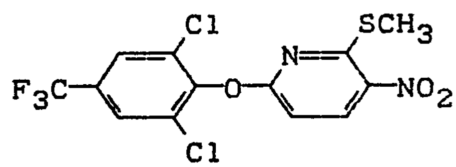

Verfahren (a)

2,9 g (0,015 Mol) 2,6-Dichlor-4-trifluormethyl-phenol werden in 50 ml Dimethylsulfoxid bei Raumtemperatur mit 0,7 g (0,0125 Mol) Kaliumhydroxidpulver versetzt und 1 Stunde nachgerührt; dann werden 2,4 g (0,015 Mol) 6-Chlor-3-nitro-2-methylthio-pyridin dazu gegeben und 6 Stunden bei 90 °C gerührt. Man dampft den Ansatz ein und nimmt den Rückstand in Methylenchlorid auf. Die Methylenchloridlösung wird mit 1N-Natronlauge und Wasser gewaschen, über Natriumsulfat getrocknet, filtriert und das Filtrat eingeengt. Das zurückbleibende Öl kristallisiert nach Verreiben mit Ethanol.

Man erhält 1,7 g (37 % der Theorie) 2-Methylthio-3-nitro-6-(2,6-dichlor-4-trifluormethyl-phenoxy)-pyridin vom Schmelzpunkt 142 °C.

Beispiel 5

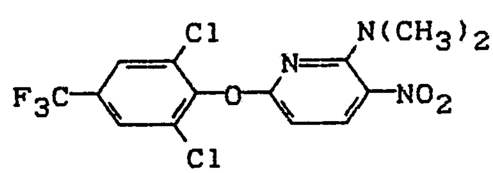

Verfahren (a)

17,4 g (0,075 Mol) 4-Trifluormethyl-2,6-dichlor-phenol werden mit 10,4 g (0,075 Mol) wasserfreiem Kaliumcarbonat in 200 ml Dimethylsulfoxid 1 Stunde bei 60 - 70 °C gerührt. Nach dem Abkühlen auf Raumtemperatur werden 10 g (0,050 Mol) 6-Chlor-2-dimethylamino-3-nitropyridin zugegeben und 3 Tage bei 120 - 140 °C gerührt. Der Ansatz wird in 200 ml Wasser eingerührt und die wäßrige Phase zweimal mit je 150 ml Methylenchlorid extrahiert. Die vereinigten Methylenchloridphasen werden über Natriumsulfat getrocknet, filtriert und eingedampft. Der verbleibende Rückstand wird durch Chromatographie mit Methylenchlorid über eine Kieselgelsäule gereinigt.

Man erhält 1,8 g (9 % der Theorie) 2-Dimethylamino-3-nitro-6-(2,6-dichlor-4-trifluormethyl-phenoxy)-pyridin in Form gelber Kristalle vom Schmelzpunkt 103 - 106 °C.

Beispiel 6

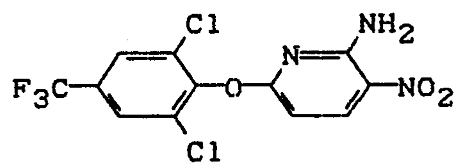

Verfahren (a)

3,2 g (0,014 Mol) 2,6-Dichlor-4-trifluormethyl-phenol, 1,4 g (0,0125 Mol) 1,4-Diazabicyclo-[2,2,2]-octan und 2,2 g (0,0125 Mol) 2-Amino-3-nitro-6-chlor-pyridin werden in 50 ml Chloroform 3 Stunden bei 60 °C gerührt. Die Chloroformlösung wird zweimal mit je 50 ml Wasser gewaschen, über Natriumsulfat getrocknet, filtriert und eingeengt. Man versetzt das zurückbleibende Öl mit 100 ml heißem Ligroin, filtriert über Kieselgel und dampft das Filtrat ein. Der ölige Rückstand kristallisiert beim Abkühlen.

Man erhält 1,66 g (36 % der Theorie) 2-Amino-3-nitro-6-(2,6-dichlor-4-trifluormethyl-phenoxy)-pyridin vom Schmelzpunkt 140 °C.

Beispiel 7

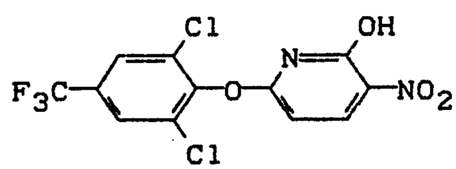

Verfahren (b)

6,6 g (0,018 Mol) 2-Amino-3-nitro-6-(2,6-dichlor-4-trifluormethyl-phenoxy)-pyridin werden in 150 ml konzentrierter Schwefelsäure gelöst, 50 ml Wasser zugegeben und die Lösung auf 0 °C abgekühlt. Dazu wird unter Rühren und Kühlen eine Lösung von 2,5 g (0,036 Mol) Natriumnitrit in 10 ml Wasser getropft. Man läßt das Reaktionsgemisch über Nacht auf Raumtemperatur kommen, verdünnt den Ansatz mit 500 ml Wasser und extrahiert die wäßrige Phase zweimal mit je 200 ml Methylenchlorid. Die vereinigten Methylenchloridphasen werden über Natriumsulfat getrocknet, filtriert und eingeengt.

Man erhält 6,1 g (92 % der Theorie) 2-Hydroxy-3-nitro-6-(2,6-dichlor-4-trifluormethylphenoxy)-pyridin vom Schmelzpunkt 177 - 182 °C.

Beispiel 8

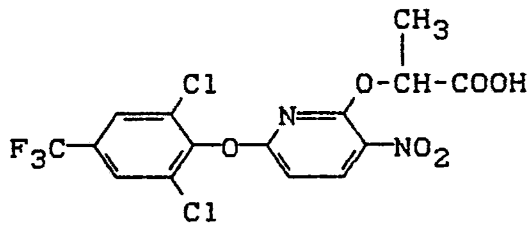

Verfahren (c)

11,4 g (0,05 Mol) 2-[3-Nitro-6-(2,6-dichlor-4-trifluormethyl-phenoxy)-pyridinyl-2-oxy)-propionsäuremethylester werden in 17 ml Ethanol verrührt und eine Lösung aus 1 g (0,025 Mol) Natriumhydroxid in 12 ml Wasser zugegeben. Man kocht 2 Stunden unter Rückfluß, wobei eine klare Lösung entsteht. Diese wird auf die Hälfte ihres Volumens eingeengt und mit Salzsäure auf pH 2 gestellt. Die ausgefallenen Kristalle werden abgesaugt, neutral gewaschen und im Exsikkator getrocknet.

Man erhält 10,6 g (96 % der Theorie) 2-[3-Nitro-6-(2,6-dichlor-4-trifluormethyl-phenoxy)-pyridinyl-2-oxy]-propionsäure vom Schmelzpunkt 170 - 176 °C.

Beispiel 9

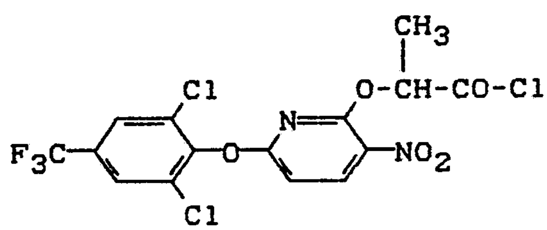

Verfahren (d)

8 g (0,0174 Mol) 2-[3-Nitro-6-(2,6-dichlor-4-trifluormethyl-phenoxy)-pyridinyl-2-oxy]-propionsäure werden in 55 ml Thionylchlorid 3 Stunden unter Rückfluß gekocht. Dann dampft man die Lösung unter vermindertem Druck ein, kristallisiert den Rückstand mit 20 ml Petrolether aus, saugt die gebildeten Kristalle ab, wäscht sie mit Petrolether und trocknet sie im Exsikkator.

Man erhält 5,5 g (69 % der Theorie) 2-[3-Nitro-6-(2,6-dichlor-4-trifluormethyl-phenoxy)-pyridinyl-2-oxy]-propionsäurechlorid vom Schmelzpunkt 94 - 98 °C.

Beispiel 10

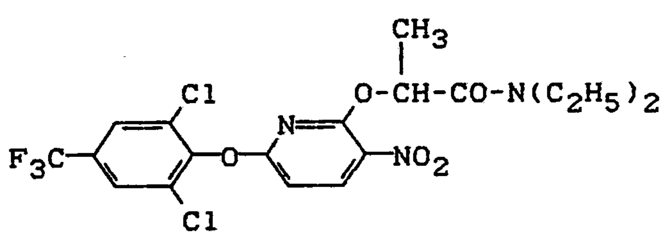

Verfahren (e)

2,3 g (0,005 Mol) 2-[3-Nitro-6-(2,6-dichlor-4-trifluormethyl-phenoxy)-pyridinyl-2-oxy]-propionsäurechlorid werden in 20 ml Methylenchlorid gelöst, 0,52 ml (0,005 Mol) Diethylamin und 0,7 ml (0,005 Mol) Triethylamin zugegeben und 3 Stunden bei 20 - 30°C gerührt. Die Methylenchloridphase wird zweimal mit je 50 ml Wasser gewaschen, über Natriumsulfat getrocknet, filtriert und eingedampft. Den öligen Rückstand kristallisiert man mit Petrolether aus, saugt die Kristalle ab, wäscht sie mit petrolether nach und trocknet sie an der Luft.

Man erhält 2,1 g (85 % der Theorie)2-[ 3-Nitro-6-(2,6-dichlor-4-trifluormethylphenoxy)-pyridin-yl-2-oxy]-propionsäurediethylamid in Form gelber Kristalle vom Schmelzpunkt 90 - 93 °C.

Beispiel 11

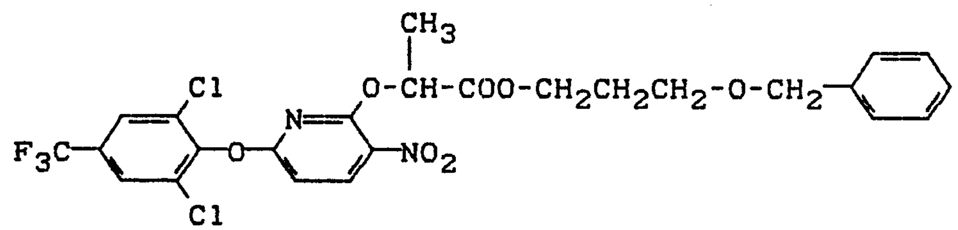

Verfahren (e)

2,3 g (0,005 Mol) 2-[3-Nitro-6-(2,6-dichlor-4-trifluormethyl-phenoxy)-pyridinyl-2-oxy]-propionsäurechlorid werden in 20 ml Methylenchlorid gelöst, 0,8 g (0,005 Mol) 3-Benzyloxy-propan-1-ol zugegeben und dann 0,7 ml (0,005 Mol) Triethylamin gelöst in 5 ml Methylenchlorid zugetropft. Das Reaktionsgemisch wird 4 Stunden bei 40 °C gerührt, mit 1N-Natronlauge und anschließend mit Wasser gewaschen, über Natriumsulfat getrocknet, filtriert und eingeengt. Das zurückbleibende Öl reinigt man durch Chromatographie über eine Kieselgelsäule, mit Methylenchlorid als Laufmittel.

Nach dem Eindampfen des Eluats verbleiben 0,44 g (15 % der Theorie) 2-[3-Nitro-6-(2,6-dichlor-4-trifluormethylphenoxy)-pyridin-yl-2-oxy]-propionsäure-3-benzyloxy-1-propylester vom Schmelzpunkt 86 - 87°C.

Analog zu den Beispielen 1 bis 11 und nach den allgemeinen Angaben zur Herstellung können die in der nachstehenden Tabelle aufgeführten Verbindungen der Formel (I) erhalten werden:

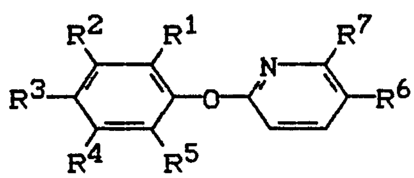
(I)

Tabelle 1: Verbindungen der Formel (I)

| Bsp. Nr. | $R^1$ | $R^2$ | $R^3$ | $R^4$ | $R^5$ | $R^6$ | $R^7$ | Schmelzpunkt (°C) |
|---|---|---|---|---|---|---|---|---|
| 12 | Cl | H | $CF_3$ | H | Cl | $NO_2$ | CN | 148 |
| 13 | Cl | H | $OCF_3$ | H | Cl | $NO_2$ | CN | 125 |
| 14 | Cl | H | $SO_2CF_3$ | H | Cl | $NO_2$ | CN | 145 |
| 15 | Cl | H | $CF_3$ | H | Cl | $NO_2$ | $CH_3$<br>\|<br>$-O-CH-COOC_2H_5$ | 101 |
| 16 | Cl | F | $CF_3$ | H | Cl | $NO_2$ | $CH_3$<br>\|<br>$-O-CH-COOCH_3$ | 119 |
| 17 | Cl | H | $OCF_3$ | H | Cl | $NO_2$ | $CH_3$<br>\|<br>$-O-CH-COOC_2H_5$ | 88 |
| 18 | Cl | H | $CF_3$ | H | Cl | $NO_2$ | $-NH-OCH_3$ | 171 |
| 19 | Cl | H | $CF_3$ | H | Cl | $NO_2$ | $-OCH_2-COOC_4H_9$ | 113 |
| 20 | Cl | H | $CF_3$ | H | Cl | $NO_2$ | Cl | 142 |
| 21 | Cl | H | $OCF_3$ | H | Cl | $NO_2$ | $SCH_3$ | 114 |
| 22 | Cl | H | $CF_3$ | H | Cl | $NO_2$ | $SC_2H_5$ | 132 |
| 23 | Cl | H | $CF_3$ | H | Cl | $NO_2$ | $SO_2CH_3$ | 167 |
| 24 | Cl | H | $OCF_3$ | H | Cl | $NO_2$ | $NH_2$ | 90 |
| 25 | Cl | H | $SO_2CF_3$ | H | Cl | $NO_2$ | $NH_2$ | 197 |
| 26 | Cl | H | $CF_3$ | H | Cl | $NO_2$ | $OCH_3$ | 101 |
| 27 | Cl | F | $CF_3$ | H | Cl | $NO_2$ | $NH_2$ | 126 |
| 28 | Cl | H | $CF_3$ | H | Cl | $NO_2$ | $NHCH_3$ | 205 |

<u>Tabelle 1</u> - Fortsetzung

| Bsp. Nr. | $R^1$ | $R^2$ | $R^3$ | $R^4$ | $R^5$ | $R^6$ | $R^7$ | Schmelzpunkt (°C) |
|---|---|---|---|---|---|---|---|---|
| 29 | $NO_2$ | H | Cl | H | Cl | $COOC_2H_5$ | H | 97 |
| 30 | Cl | H | $CF_3$ | H | Cl | $NO_2$ | $OCHF_2$ | |
| 31 | Cl | H | $CF_3$ | H | Cl | $NO_2$ | $-OCH_2CH{=}CH_2$ | |
| 32 | Cl | H | $CF_3$ | H | Cl | $NO_2$ | $-OCH_2C{\equiv}CH$ | |
| 33 | Cl | H | $CF_3$ | H | Cl | $NO_2$ | $-SCH_2COOCH_3$ | 225 |
| 34 | Cl | H | $CF_3$ | H | Cl | $NO_2$ | $-OCH_2CH_2SCH_3$ | |
| 35 | Cl | H | $CF_3$ | H | Cl | $NO_2$ | $CH_3$<br>$-OCHCOOCH_2CH_2SCH_3$ | |
| 36 | Cl | H | $CF_3$ | H | Cl | $NO_2$ | $CH_3$ $C_2H_5$<br>$-OCHCOOCH_2CH_2S$ | |
| 37 | Cl | H | $CF_3$ | H | Cl | $NO_2$ | $CH_3$ O<br>$-OCHCOOCH_2P(OCH_3)_2$ | |
| 38 | Cl | H | $CF_3$ | H | Cl | $NO_2$ | $CH_3$ $CH_3$ O<br>$-OCHCOOCH—P(OCH_3)_2$ | |
| 39 | Cl | H | $CF_3$ | H | Cl | $NO_2$ | $CH_3$<br>$-OCH-CO-NHC_2H_5$ | |
| 40 | Cl | H | $CF_3$ | H | Cl | $NO_2$ | $CH_3$<br>$-OCH-CO-NH-SO_2CH_3$ | |
| 41 | Cl | H | $CF_3$ | H | Cl | $NO_2$ | $CH_3$<br>$-OCH-COOCH_2H_2OCH_3$ | |

<u>Tabelle 1</u> - Fortsetzung

| Bsp. Nr. | $R^1$ | $R^2$ | $R^3$ | $R^4$ | $R^5$ | $R^6$ | $R^7$ | Schmelzpunkt (°C) |
|---|---|---|---|---|---|---|---|---|
| 42 | Cl | H | $CF_3$ | H | Cl | $NO_2$ | 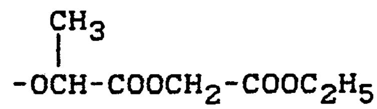 | |
| 43 | Cl | H | $CF_3$ | H | Cl | $NO_2$ | 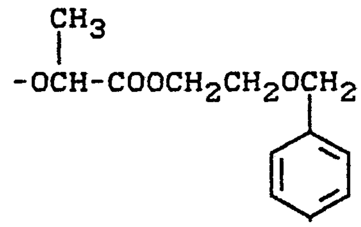 | |
| 44 | Cl | H | $CF_3$ | H | Cl | $NO_2$ | 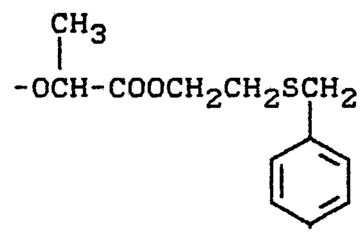 | |
| 45 | Cl | H | $CF_3$ | H | Cl | $NO_2$ | 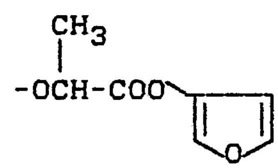 | |
| 46 | Cl | H | Cl | H | $NO_2$ | COOH | H | 125 |
| 47 | Cl | H | $OCF_3$ | H | Cl | $NO_2$ | $-NHOCH_3$ | 125 |
| 48 | Cl | F | $CF_3$ | H | Cl | $NO_2$ | $-NHOCH_3$ | 147 |
| 49 | Cl | H | $CF_3$ | H | Cl | $NO_2$ | 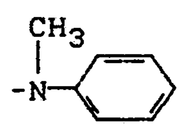 | 137 |
| 50 | Cl | H | $CF_3$ | H | Cl | $NO_2$ | $-NH-COCH_3$ | 153 |
| 51 | Cl | H | $OCF_3$ | H | Cl | $NO_2$ | $-OCH_2COOC_4H_9$ | 92 |

<u>Tabelle 1</u> - Fortsetzung

| Bsp. Nr. | $R^1$ | $R^2$ | $R^3$ | $R^4$ | $R^5$ | $R^6$ | $R^7$ | Schmelzpunkt (°C) |
|---|---|---|---|---|---|---|---|---|
| 52 | Cl | F | $CF_3$ | H | Cl | $NO_2$ | $-OCH_2COOC_4H_9$ | 66 |
| 53 | Cl | H | $CF_3$ | H | Cl | $NO_2$ | $-OCH_2COOCH_3$ | 101 |
| 54 | Cl | H | $CF_3$ | H | Cl | $NO_2$ | $-OCH_2CONH_2$ | |
| 55 | Cl | H | $CF_3$ | H | Cl | $NO_2$ | $-SO_2C_2H_5$ | 170 |
| 56 | Cl | H | $CF_3$ | H | Cl | $NO_2$ | $-O-\overset{S}{\overset{\|}{P}}(OC_2H_5)_2$ | 130 |
| 57 | Cl | H | $OCF_3$ | H | Cl | $NO_2$ | $-OCH_3$ | 78 |
| 58 | Cl | F | $CF_3$ | H | Cl | $NO_2$ | $-NHCH_2COOC_2H_5$ | 144 |
| 59 | Cl | H | $CF_3$ | H | Cl | $NO_2$ | $-SOC_2H_5$ | 140 |
| 60 | Cl | H | $CF_3$ | H | Cl | $NO_2$ | $-CH_3$ | 123 |
| 61 | Cl | H | $CF_3$ | H | Cl | $NO_2$ | $-NH-\underset{CH_3}{\underset{\|}{CH}}-COOC_2H_5$ | 110 |
| 62 | Cl | H | $OCF_3$ | H | Cl | $NO_2$ | $-NH-\underset{CH_3}{\underset{\|}{CH}}-COOC_2H_5$ | 88 |
| 63 | Cl | H | $CF_3$ | H | Cl | $NO_2$ | $-S-CH_2CH_2-COOCH_3$ | 115 |
| 64 | Cl | H | $OCF_3$ | H | Cl | $NO_2$ | $-CH_3$ | 108 |
| 65 | Cl | H | $OCF_3$ | H | Cl | $NO_2$ | $-S-CH_2-COOCH_3$ | 118 |
| 66 | Cl | H | $CF_3$ | H | Cl | $NO_2$ | $-SOCH_3$ | 190 |
| 67 | Cl | H | $CF_3$ | H | Cl | $NO_2$ | $-O-\overset{S}{\overset{\|}{P}}(OCH_3)_2$ | 155 |

Tabelle 1 - Fortsetzung

| Bsp. Nr. | $R^1$ | $R^2$ | $R^3$ | $R^4$ | $R^5$ | $R^6$ | $R^7$ | Schmelzpunkt (°C) |
|---|---|---|---|---|---|---|---|---|
| 68 | Cl | H | $CF_3$ | H | Cl | $NO_2$ | 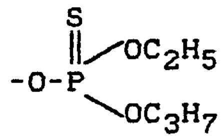 | 128 |
| 69 | Cl | H | $CF_3$ | H | Cl | $NO_2$ | $-N(C_2H_5)_2$ | |
| 70 | Cl | F | $CF_3$ | H | Cl | $NO_2$ | Cl | 91 |
| 71 | Cl | H | $OCF_3$ | H | Cl | $NO_2$ | $-N(CH_3)_2$ | 91 |
| 72 | Cl | H | $OCF_3$ | H | Cl | $NO_2$ | $-SC_2H_5$ | |
| 73 | Cl | H | $C(CH_3)_3$ | H | Cl | $NO_2$ | $NH_2$ | 117 |
| 74 | Cl | H | $SCF_3$ | H | Cl | $NO_2$ | $NH_2$ | 148 |
| 75 | Cl | H | $SCF_3$ | H | Cl | $NO_2$ | $-SCH_3$ | 139 |
| 76 | Cl | H | $CF_3$ | H | Cl | $NO_2$ | $-S-CH_2-$ 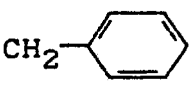 | 115 |
| 77 | Cl | H | $OCF_3$ | H | Cl | $NO_2$ | $-SOCH_3$ | 102 |
| 78 | Cl | F | $CF_3$ | H | Cl | $NO_2$ | $-S-CH_2-COOCH_3$ | 118 |
| 79 | Cl | H | $CF_3$ | H | Cl | $NO_2$ | -NH-NH- 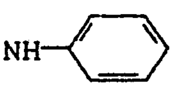 | 178 |
| 80 | Cl | H | $CF_3$ | H | Cl | $NO_2$ | 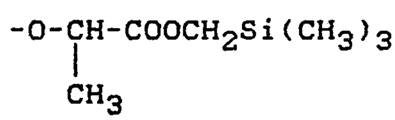 | |
| 81 | Cl | F | $CF_3$ | H | Cl | $NO_2$ | $-S-CH_2-COOC_2H_5$ | |
| 82 | Cl | H | $OCF_3$ | H | Cl | $NO_2$ | $-SO_2CH_3$ | 119 |
| 83 | Cl | H | $CF_3$ | H | Cl | $NO_2$ | $-O-CH_2-COOH$ | 175 |

Tabelle 1 - Fortsetzung

| Bsp. Nr. | $R^1$ | $R^2$ | $R^3$ | $R^4$ | $R^5$ | $R^6$ | $R^7$ | Schmelzpunkt (°C) |
|---|---|---|---|---|---|---|---|---|
| 84 | Cl | H | $CF_3$ | H | Cl | $NO_2$ | $-O-CH_2-CO-CH_3$ | 108 |
| 85 | Cl | H | $CF_3$ | H | Cl | $NO_2$ | $-NH-NH-CO-CH_3$ | 235 |
| 86 | Cl | H | $CF_3$ | H | Cl | $NO_2$ | $-NH-NH_2$ | 150 |
| 87 | Cl | F | $CF_3$ | H | Cl | $NO_2$ | $-S-CH_2CH_2-COOCH_3$ | 121 |
| 88 | Cl | F | $CF_3$ | H | Cl | $NO_2$ | $-OCH_2COOCH_3$ | 82 |
| 89 | Cl | H | $CF_3$ | H | Cl | $NO_2$ | $-OCH_2-C(CH_3)=N-OH$ | 118 |
| 90 | Cl | H | $CF_3$ | H | Cl | $NO_2$ | $-OCH_2-C(CH_3)=N-O-CH_2(COOC_2H_5)$ | 97 |
| 91 | Cl | F | $CF_3$ | H | Cl | $NO_2$ | $-OCH_2-CO-CH_3$ | |
| 92 | Cl | F | $CF_3$ | H | Cl | $NO_2$ | $-OCH_2-COOC_2H_5$ | |
| 93 | Cl | H | $OCF_3$ | H | Cl | $NO_2$ | $-OCH_2-COOC_2H_5$ | 102 |
| 94 | Cl | H | $OCF_3$ | H | Cl | $NO_2$ | $-OCH_2-COOCH_3$ | 90 |
| 95 | Cl | H | $CF_3$ | H | Cl | $NO_2$ | $-CH(COOC_2H_5)_2$ | 108-11 |

Nachstehend werden - in Ergänzung der Beispiele 1 bis 11 - einige weitere erfindungsgemäße Herstellungsverfahren exemplarisch beschrieben:

Herstellung der vorausgehend als Beispiel 50 aufgeführten Verbindung

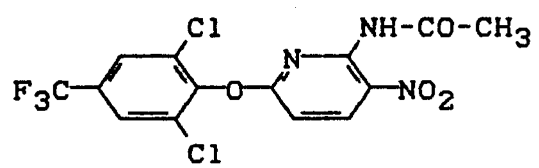

Verfahren (f)

Eine Mischung aus 1,8 g (0,005 Mol) 2-Amino-3-nitro-6-(2,6-dichlor-4-trifluormethyl-phenoxy)-pyridin (vgl. Beispiel 6), 0,95 g (0,01 Mol) Acetanhydrid, 15 ml Essigsäure und einigen Tropfen konz. Schwefelsäure wird 4 Stunden bei 60 °C gerührt. Nach dem Abkühlen wird mit Natriumhydrogencarbonat neutralisiert und mit Methylenchlorid extrahiert. Die Methylenchlorid-Phase wird mit wäßriger Natriumhydrogencarbonat-Lösung gewaschen mit Magnesiumsulfat getrocknet und filtriert. Vom Filtrat wird das Lösungsmittel im Wasserstrahlvakuum abdestilliert.

Man erhält 1,7 g (83 % der Theorie) 2-Acetylamino-3-nitro-6-(2,6-dichlor-4-trifluormethyl-phenoxy)-pyridin als kristallinen Rückstand vom Schmelzpunkt 153 °C

Herstellung der vorausgehend als Beispiel 23 aufgeführten Verbindung

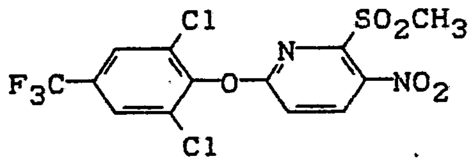

Verfahren (g)

Eine Mischung aus 2,0 g (0,005 Mol) 2-Methylthio-3-nitro-6-(2,6-dichlor-4-trifluormethyl-phenoxy)-pyridin (vgl. Beispiel 4), 0,6 g (0,013 Mol) Ameisensäure, 1,0 g Perhydrol-35 %ig (entsprechend 0,01 Mol $H_2O_2$), 30 ml Methylenchlorid und einer Spatelspitze Ammoniummolybdat wird 2 Tage bei 20 °C gerüht. Man wäscht dann mit wäßriger Natriumhydrogensulfit-Lösung, trocknet mit Magnesiumsulfat und filtriert. Vom Filtrat wird das Lösungsmittel im Wasserstrahlvakuum abdestilliert, der Rückstand durch Verreiben mit Ethanol zur Kristallisation gebracht und das Produkt durch Absaugen isoliert.

Man erhält 1,6 g (74 % der Theorie) 2-Methylsulfonyl-3-nitro-6-(2,6-dichlor-4-trifluormethyl-phenoxy)-pyridin vom Schmelzpunkt 167 °C.

Herstellung der vorausgehend als Beispiel 26 aufgeführten Verbindung

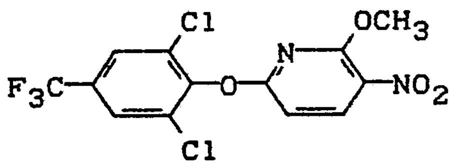

Verfahren (h)

Eine Mischung aus 1,9 g (0,005 Mol) 2-Chlor-3-nitro-6-(2,6-dichlor-4-trifluormethyl-phenoxy)-pyridin (vgl. Beispiel 20), 1,0 g einer 40 %igen methanolischen Natriummethylat-Lösung (entsprechend 0,005 Mol $NaOCH_3$) und 20 ml Methanol wird 15 Stunden bei 20 °C gerührt. Dann wird mit Wasser verdünnt und mit Methylenchlorid extrahiert. Die Methylenchlorid-Phase wird mit Wasser gewaschen, mit Magnesiumsulfat getrocknet und filtriert. Vom Filtrat wird das Lösungsmittel im Wasserstrahlvakuum abdestilliert, der Rückstand durch Verreiben mit Ethanol zur Kristallisation gebracht und durch Absaugen isoliert.

Man erhält 1,0 g (52 % der Theorie) 2-Methoxy-3-nitro-6-(2,6-dichlor-4-trifluormethyl-phenoxy)-pyridin vom Schmelzpunkt 101 °C.

Herstellung der vorausgehend als Beispiel 20 aufgeführten Verbindung

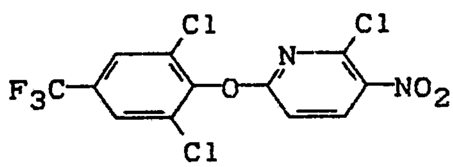

Verfarhen (i)

4,0 g (0,058 Mol) Natriumnitrit werden bei 20 °C unter Rühren zu einer Mischung aus 19,0 g (0,050 Mol) 2-Amino-3-nitro-6-(2,6-dichlor-4-trifluormethyl-phenoxy)-pyridin (vgl. Beispiel 6), 200 ml konz. Salzsäure und 200 ml Essigsäure gegeben und das Reaktionsgemisch wird 2 Stunden gerührt. Nach Zugabe von 6 g Kupfer wird das Rühren noch 15 Stunden fortgesetzt. Dann wird mit Methylenchlorid geschüttelt, die organische Phase abgetrennt, mit Wasser gewaschen, mit Magnesiumsulfat getrocknet und

filtriert. Vom Filtrat wird das Lösungsmittel im Wasserstrahlvakuum abdestilliert.

Man erhält 8,1 g (42 % der Theorie) 2-Chlor-3-nitro-6-(2,6-dichlor-4-trifluormethyl-phenoxy)-pyridin vom Schmelzpunkt 142 °C.

Herstellung der vorausgehend als Beispiel 80 aufgeführten Verbindung

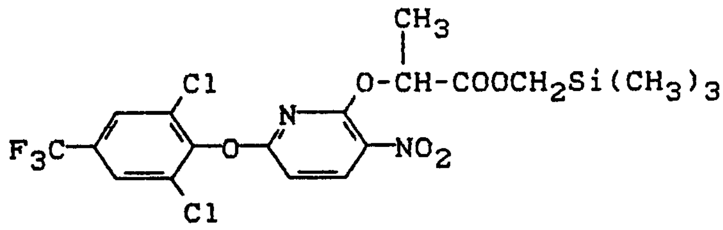

Verfahren (j)

0,8 g (0,006 Mol) Chlormethyl-trimethylsilan werden bei 20 °C unter Rühren zu einer Mischung aus 2,2 g (0,005 Mol) 2-(1-Carboxy-ethoxy)-3-nitro-6-(2,6-dichlor-4-trifluormethyl-phenoxy)-pyridin, 0,9 g (0,006 Mol) 1,8-Diazabicyclo-[5.4.0]-undecen (DBU) und 50 ml Aceton gegeben und das Reaktionsgemisch wird 15 Stunden unter Rückfluß zum Sieden erhitzt. Dann wird eingeengt, der Rückstand in Methylenchlorid aufgenommen, mit 2N-Salzsäure gewaschen, mit Magnesiumsulfat getrocknet und filtriert. Vom Filtrat wird das Lösungsmittel im Wasserstrahlvakuum abdestilliert.

Man erhält 1,3 g (49 % der Theorie) 2-(1-Trimethylsilylmethoxycarbonyl-ethoxy)-3-nitro-6-(2,6-dichlor-4-trifluormethyl-phenoxy)-pyridin als amorphen Rückstand.

Ausgangsstoffe der Formel (II)

Beispiel II-1

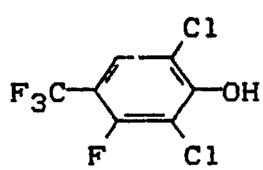

50 g (0,9 Mol) Kaliumhydroxid-Pulver werden bei 20 °C portionsweise zu einer Mischung aus 75 g (0,3 Mol) 3,5-Dichlor-2,4-difluor-benzotrifluorid, 400 ml tert-Butanol und 50 ml Wasser unter Rühren gegeben. Das Reaktionsgemisch wird 6 Stunden bei 50 °C gerührt, abgekühlt, auf 600 ml Wasser gegossen und unter Kühlen mit Salzsäure angesäuert. Dann wird mit Methylenchlorid extrahiert, die Methylenchlorid-Phase mit Magnesiumsulfat getrocknet und filtriert. Vom Filtrat wird das Lösungsmittel an einem Rotationsverdampfer abdestilliert und das zurückbleibende Produkt durch Destillation über eine Drehbandkolonne gereinigt.

Man erhält 48 g (64 % der Theorie) 2,4-Dichlor-3-fluor-6-trifluormethyl-phenol vom Siedepunkt 78 - 80 °C (14 mbar) und Brechungsindex $n_D^{20}$ = 1,4870.

Analog Beispiel II-1 und nach den allgemeinen Angaben zur Herstellung können folgende Verbindungen der Formel (II) hergestellt werden:

II-2 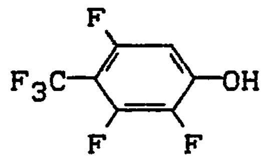 Kp: 50 - 51 °C (16 mbar)
$n_D^{20}$ = 1,4210

II-3 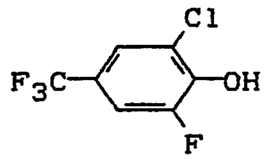 Kp: 65 - 67 °C (18 mbar)
$n_D^{20}$ = 1,4710

II-4 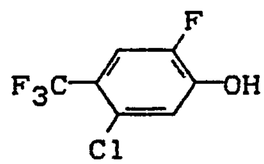 Kp: 63 - 64 °C (20 mbar)

II-5 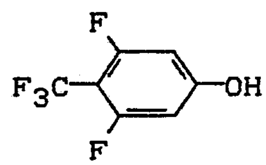 Kp: 52 - 53 °C (18 mbar)

II-6 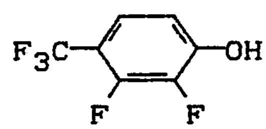 Kp: 54 °C (18 mbar)

II-7 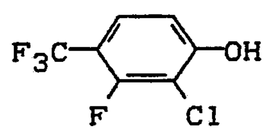 Kp: 67 - 68 °C (20 mbar)

II-8 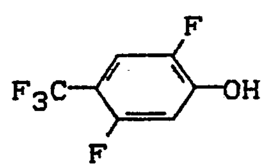 Kp: 50 - 52 °C (16 mbar)

Anwendungsbeispiele

In den folgenden Anwendungsbeispielen wird die nachstehend aufgeführte Verbindung als Vergleichssubstanz herangezogen:

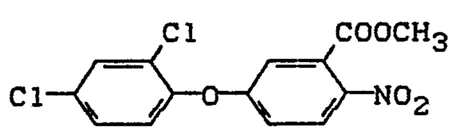 (A)

3-(2,4-Dichlor-phenoxy)-6-nitrobenzoesäure-methylester
(bekannt aus US-PS 36 52 645 und US-PS 3 776 715/Beispiel 5))

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen beispielsweise die Wirkstoffe gemäß den Herstellungsbeispielen (1), (8), (9) und (11) bei der Bekämpfung von Unkräutern, wie z.B. Abutilon, Galinsoga, Matricaria, Sinapis, Panicum und Setaria eine deutlisch stärkere Wirkung als die Vergleichssubstanz (A).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebe Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Wirkstoffe gemäß den Herstellungsbeispielen (1), (6), (8) und (9) bei der Bekämpfung von Unkräutern, wie z. B. Chenopodium, Datura, Galinsoga, Ipomoea, Portulak, Echinochloa und Setaria eine deutlich stärkere Wirkung als die Vergleichssubstanz (A).

Weiter zeigen beispielsweise die Wirkstoffe gemäß den Herstellungsbeispielen (26), (27), (33), 58), (61), (63), (83), (84) und (89) bei guter Weizenverträglichkeit erheblich stärkere Wirkung gegen Unkräuter als die Vergleichssubstanz (A).

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächschaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:
0 kein Austrocknen der Blätter, kein Blattfall

+ leichtes Austrocknen der Blätter, geringer Blattfall
+ + starkes Austrocknen der Blätter, starker Blattfall
+ + + sehr starkes Austrocknen der Blätter, sehr starker Blattfall

In diesem Test zeigen beispielsweise die Wirkstoffe gemäß den Herstellungsbeispielen (1) und (8) ein sehr starkes Austrocknen der Blätter und sehr starken Blattfall.

## Ansprüche

1. Substituierte Phenoxypyridine der Formel (I)

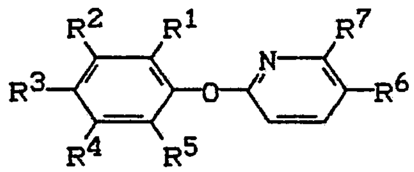

(I)

in welcher
$R^1$ für Wasserstoff, Halogen, Cyano, Nitro oder für einen gegebenenfalls durch Halogen substituierten Rest aus der Reihe Alkyl, Alkoxy, Alkylthio, Alkylsulfinyl und Alkylsulfonyl steht,
$R^2$ für Wasserstoff, Halogen oder Alkyl steht,
$R^3$ für Wasserstoff, Halogen, Cyano, Nitro oder für einen gegebenenfalls durch Halogen substituierten Rest aus der Reihe Alkyl, Alkoxy, Alkylthio, Alkylsulfinyl und Alkylsulfonyl steht,
$R^4$ für Wasserstoff oder Halogen steht und
$R^5$ für Wasserstoff oder Halogen steht
-mit der Maßgabe, daß wenigstens drei der Substituenten $R^1$ bis $R^5$ von Wasserstoff verschieden sind und wenigstens einer der Reste $R^1$ bis $R^5$ von Wasserstoff und Halogen verschieden ist -
in welcher weiter
$R^6$ für Wasserstoff, Cyano, Nitro, Halogen, Carboxy, Carbamoyl oder Alkoxycarbonyl steht und
$R^7$ für Wasserstoff, Halogen, Cyano, Nitro, Formyl, Hydroxy, Trialkylsilyloxy, Alkylaminocarbonyloxy, Dialkoxy(thio)phosphoryloxy, oder für $-O^{\ominus}M^{\oplus}$ steht, wobei
$M^{\oplus}$ für ein Ammoniumion, ein Alkylammoniumion, ein Alkalimetallion oder ein Erdalkalimetallionenäquivalent steht oder
$R^7$ für einen gegebenenfalls durch Halogen, Cyano, Nitro, Carboxy, Carbamoyl, Amino, Phenyl, Alkylcarbonyl, Alkylamino, Dialkylamino, Hydroxy, Alkoxy, Alkylthio, Alkylaminocarbonyl, Alkoxycarbonyl und/oder Alkoxycarbonylalkoxy substituierten Rest aus der Reihe Alkyl, Alkenyl, Alkinyl, Alkoxy, Alkenyloxy, Alkinyloxy, Alkylthio, Alkenylthio, Alkinylthio, Alkylsuflinyl und Alkylsulfonyl steht oder
$R^7$ für den Rest

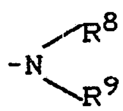

steht, worin
$R^8$ für Wasserstoff oder Alkyl steht und
$R^9$ für Wasserstoff, Hydroxy, Amino, Alkylamino, Dialkylamino, Arylamino, Alkylcarbonylamino, Arylcarbonylamino, Alkylsulfonylamino, gegebenenfalls im Arylteil durch Alkyl substituiertes Arylsulfonylamino, Cyano, Alkyl, Alkoxy, Halogenalkyl, Alkoxycarbonyl, Alkylcarbonyl, Arylcarbonyl, Aryloxycarbonyl, Alkylaminocarbonyl, Arylaminocarbonyl, Alkoxycarbonylalkyl, Aralkyl, Alkylsulfonyl, Arylsulfonyl oder für gegebenenfalls durch Halogen, Cyano, Nitro, Alkyl, Alkoxy, Halogenalkyl und/oder Halogenalkoxy substituiertes Aryl steht, oder
$R^7$ für den Rest $-O-\overset{R^{10}}{\overset{|}{C}H}-CO-R^{11}$ steht, worin
$R^{10}$ für Wasserstoff oder Alkyl steht und
$R^{11}$ für Halogen, Hydroxy, Amino, Alkylamino, Cyanamino, Dialkylamino, Alkylsulfonylamino, Arylsulfonylamino, Hydroxyamino, Alkoxyamino, Hydrazino, Alkylsulfonylhydrazino, Arylsulfonylhydrazino oder für den Rest -Q-$R^{12}$ steht, worin
Q für Sauerstoff oder Schwefel steht und
$R^{12}$ für gegebenenfalls durch Halogen substituierte Reste aus der Reihe Alkyl, Alkoxyalkyl, Alkylthioalkyl, Alkylsulfinylalkyl, Alkylsulfonylalkyl, Aryloxyalkyl, Trialkylsilylalkyl, Arylthioalkyl, Aralkoxyalkyl, Alkoxycarbonylalkyl, Alkylaminocarbonylalkyl, Aralkylthioalkyl, Aralkyl, Azolylalkyl oder für ein Ammonium-, Alkylammonium-, Alkalimetall-oder Erdalkalimetall-äquivalent steht, oder
$R^7$ für den Rest

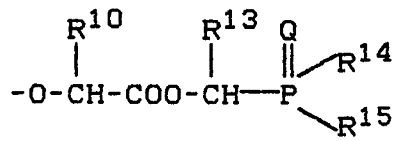

steht,
worin
$R^{10}$ für Wasserstoff oder Alkyl steht,
$R^{13}$ für Wasserstoff, Alkyl, Aryl, Furyl, Thienyl oder Pyridyl steht,
$R^{14}$ für Alkyl oder Alkoxy steht,
$R^{15}$ für Alkoxy steht und
Q für Sauerstoff oder Schwefel steht, oder
$R^7$ für den Rest

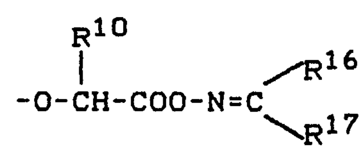

steht, worin
$R^{10}$ für Wasserstoff oder Alkyl steht,
$R^{16}$ für Wasserstoff oder Alkyl steht und
$R^{17}$ für Alkyl oder Aryl steht, oder
$R^7$ für den Rest -S(O)$_n$-Y steht, worin
n für die Zahlen 0, 1 oder 2 steht und
Y für Alkoxy, Alkenoxy, Alkinoxy, Aralkoxy, Aryloxy oder für Amino, Alkylamino, Alkenylamino, Alkinylamino, Aralkylamino, Arylamino, Dialkylamino, Cyanamino, Guanidino, Hydroxyamino, Alkoxyamino, Hydrazino, Alkylhydrazino, Arylhydrazino, Alkylcarbonylhydrazino, Alkoxycarbonylhydrazino, Alkylsulfonylhydrazino oder Arylsulfonylhydrazino steht, oder
$R^7$ für den Rest

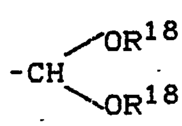

steht, worin die beiden Reste $R^{18}$ einzeln für Alkyl oder zusammen für Alkandiyl ("Alkylen") stehen, oder
$R^7$ für den Rest $-(O)_p-(A)_q-\overset{R^{19}}{\overset{|}{C}}=N-R^{20}$ steht, worin
A für Alkandiyl (Alkylen) steht,
p und q jeweils für die Zahlen 0 oder 1 stehen,
$R^{19}$ für Wasserstoff, Alkyl, Alkoxy oder Alkylamino steht und
$R^{20}$ für Wasserstoff, Alkyl, Aryl, Hydroxy, für gegebenenfalls durch Alkoxycarbonyl substituiertes Alkoxy, für Alkenyloxy, Alkinyloxy oder Aralkoxy steht, oder
$R^7$ für den Rest

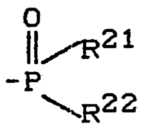

steht, worin
$R^{21}$ für Alkyl oder Alkoxy steht und
$R^{22}$ für Alkoxy steht, oder
$R^7$ für den Rest -$(CH_2)_m$-Z oder für den Rest

$R^{10}$
-O- CH – COO-$(CH_2)_m$-Z steht, worin
m für die Zahlen 0, 1 oder 2 steht,
$R^{10}$ für Wasserstoff oder Alkyl steht, und
Z für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl, Pyrimidinyl und Pyridazinyl steht.

2. Substituierte Phenoxypyridine der Formel (I), gemäß Anspruch 1, in welcher
$R^1$ für Wasserstoff, Fluor, Chlor, Brom, Cyano, Nitro oder für einen gegebenenfalls durch Fluor und/oder Chlor substituierten Rest aus der Reihe $C_1$-$C_2$-Alkyl, $C_1$-$C_2$-Alkoxy, $C_1$-$C_2$-Alkylthio, $C_1$-$C_2$-Alkylsulfinyl und $C_1$-$C_2$-Alkylsulfonyl steht,
$R^2$ für Wasserstoff, Fluor, Chlor, Brom oder $C_1$-$C_2$-Alkyl steht,
$R^3$ für Wasserstoff, Fluor, Chlor, Brom, Cyano, Nitro oder für einen gegebenenfalls durch Fluor und/oder Chlor substituierten Rest aus der Reihe $C_1$-$C_4$-Alkyl, $C_1$-$C_4$-Alkoxy, $C_1$-$C_4$-Alkylthio, $C_1$-$C_4$-Alkylsulfinyl und $C_1$-$C_4$-Alkylsulfonyl steht,
$R^4$ für Wasserstoff, Fluor, Chlor oder Brom steht und
$R^5$ für Wasserstoff, Fluor, Chlor oder Brom steht
-mit der Maßgabe, daß wenigstens drei der Substituenten $R^1$ bis $R^5$ von Wasserstoff verschieden sind und wenigstens einer der Reste $R^1$ bis $R^5$ von Wasserstoff, Fluor, Chlor und Brom verschieden ist -
in welcher weiter
$R^6$ für Wasserstoff, Cyano, Nitro, Fluor, Chlor, Brom, Carboxy, Carbamoyl oder $C_1$-$C_4$-Alkoxycarbonyl steht und
$R^7$ für Wasserstoff, Fluor, Chlor, Brom, Cyano, Nitro, Formyl, Hydroxy, Trimethylsilyloxy, $C_1$-$C_4$-Alkylamino-Carbonyloxy, Di-($C_1$-$C_4$-alkoxy)-(thio)phosphoryloxy oder für -$O^{\ominus}M^{\oplus}$ steht, wobei
$M^{\oplus}$ für ein Ammoniumion, ein $C_1$-$C_4$-Alkylammoniumion, ein Natriumion, ein Kaliumion oder ein Calciumionenäquivalent steht, oder
$R^7$ für gegebenenfalls durch Fluor, Chlor, Brom, Cyano, Nitro, Carboxy, Carbamoyl, Amino, Phenyl, $C_1$-$C_4$-Alkyl-carbonyl, $C_1$-$C_4$-Alkylamino, Di-($C_1$-$C_4$-alkyl)-amino, Hydroxy, $C_1$-$C_4$-Alkoxy, $C_1$-$C_4$-Alkylthio, $C_1$-$C_4$-Alkylaminocarbonyl, $C_1$-$C_4$-Alkoxy-carbonyl und/oder $C_1$-$C_4$-Alkoxy-carbonyl-$C_1$-$C_4$-alkoxy substituiertes $C_1$-$C_6$-Alkyl, für gegebenenfalls durch Fluor, Chlor, Brom, Cyano, Nitro, Carboxy, Carbamoyl und/oder $C_1$-$C_4$-Alkoxycarbonyl substituiertes $C_2$-$C_6$-Alkenyl, für gegebenenfalls durch Fluor, Chlor, Brom, Cyano und/oder $C_1$-$C_4$-Alkoxycarbonyl substituiertes $C_2$-$C_6$-Alkinyl, für gegebenenfalls durch Fluor, Chlor, Cyano, Di-($C_1$-$C_4$-alkyl)-amino, Hydroxy, $C_1$-$C_4$-Alkylcarbonyl, $C_1$-$C_4$-Alkoxy und/oder $C_1$-$C_4$-Alkylthio substituiertes $C_1$-$C_4$-Alkoxy, für gegebenenfalls durch Fluor, Chlor, Cyano und/oder $C_1$-$C_4$-Alkoxy-carbonyl substituiertes $C_2$-$C_4$-Alkenyloxy, für gegebenenfalls durch Fluor, Chlor, Brom, Cyano und/oder $C_1$-$C_4$-Alkoxycarbonyl substituiertes $C_2$-$C_4$-Alkinyloxy, für gegebenenfalls durch Fluor, Chlor, Brom, Cyano, Carboxy, Carbamoyl, $C_1$-$C_4$-Alkoxy, $C_1$-$C_4$-Alkylthio und/oder $C_1$-$C_4$-Alkoxy-carbonyl substituiertes $C_1$-$C_4$-Alkylthio, für gegebenenfalls durch Fluor, Chlor und/oder Brom substituiertes $C_2$-$C_4$-Alkenylthio, für jeweils gegebenenfalls durch Fluor, Chlor und/oder Brom substituiertes $C_2$-$C_4$-Alkinylthio, $C_1$-$C_4$-Alkylsulfinyl oder $C_1$-$C_4$-Alkylsulfonyl steht
oder
$R^7$ für den Rest

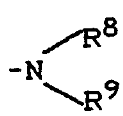

steht, worin

$R^8$ für Wasserstoff oder $C_1$-$C_4$-Alkyl steht und

$R^9$ für Wasserstoff, Hydroxy, Amino, $C_1$-$C_6$-Alkylamino, Dimethylamino, Phenylamino, $C_1$-$C_4$-Alkyl-carbonylamino, Phenylcarbonylamino, $C_1$-$C_4$-Alkylsulfonylamino, Phenylsulfonylamino, 4-Methyl-phenylsulfonylamino, Cyano, $C_1$-$C_4$-Alkyl, $C_1$-$C_4$-Alkoxy, $C_1$-$C_4$-Fluoralkyl, $C_1$-$C_4$-Alkoxycarbonyl, $C_1$-$C_4$-Alkyl-carbonyl, Benzoyl, Phenoxycarbonyl, $C_1$-$C_4$-Alkylaminocarbonyl, Phenylaminocarbonyl, $C_1$-$C_4$-Alkoxy-carbonyl-$C_1$-$C_2$-alkyl, Benzyl, $C_1$-$C_4$-Alkylsulfonyl, Phenylsulfonyl oder für gegebenenfalls durch Fluor, Chlor, Brom, Cyano, Nitro, $C_1$-$C_4$-Alkyl, $C_1$-$C_4$-Alkoxy, $C_1$-$C_4$-Halogenalkyl und/oder $C_1$-$C_4$-Halogenalkoxy substituiertes Phenyl steht,

oder

$R^7$ für den Rest $-O-\overset{R^{10}}{\overset{|}{C}}H-CO-R^{11}$ steht, worin

$R^{10}$ für Wasserstoff oder $C_1$-$C_4$-Alkyl steht und

$R^{11}$ für Chlor, Hydroxy, Amino, $C_1$-$C_4$-Alkylamino, Cyanamino, Di-($C_1$-$C_4$-alkyl)-amino, $C_1$-$C_4$-Alkylsulfonylamino, Phenylsulfonylamino, Hydroxyamino, $C_1$-$C_4$-Alkoxyamino, Hydrazino, $C_1$-$C_4$-Alkylsulfonyl-hydrazino, Phenylsulfonylhydrazino oder für den Rest -Q-$R^{12}$ steht, worin

Q für Sauerstoff oder Schwefel steht und

$R^{12}$ für gegebenenfalls durch Fluor und/oder Chlor substituierte Reste aus der Reihe $C_1$-$C_6$-Alkyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_4$-alkyl, Trimethylsilyl-$C_1$-$C_4$-alkyl, Phenylthio-$C_1$-$C_4$-alkyl, Benzyloxy-$C_1$-$C_4$-alkyl, $C_1$-$C_4$-Alkoxycarbonyl-$C_1$-$C_4$-alkyl, $C_1$-$C_4$-Alkylamino-carbonyl-$C_1$-$C_4$-alkyl, Benzylthio-$C_1$-$C_4$-alkyl, Benzyl, Phenethyl, Pyrazolyl-$C_1$-$C_4$-alkyl, Imidazolyl-$C_1$-$C_4$-alkyl, Triazolyl-$C_1$-$C_4$-alkyl oder für ein Ammonium-, ein $C_1$-$C_4$-Alkylammonium-, ein Natrium-, Kalium-oder Calcium-Äquivalent steht,

oder

$R^7$ für den Rest

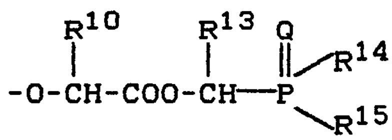

steht,

worin

$R^{10}$ für Wasserstoff oder $C_1$-$C_4$-Alkyl steht,

$R^{13}$ für Wasserstoff, $C_1$-$C_4$-Alkyl, Phenyl, Furyl, Thienyl oder Pyridyl steht,

$R^{14}$ für $C_1$-$C_4$-Alkyl oder $C_1$-$C_4$-Alkoxy steht,

$R^{15}$ für $C_1$-$C_4$-Alkoxy steht und

Q für Sauerstoff oder Schwefel steht,

oder

$R^7$ für den Rest

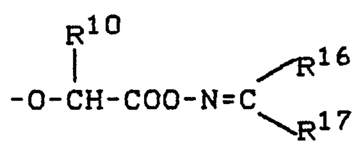

steht, worin

$R^{10}$ für Wasserstoff oder $C_1$-$C_4$-Alkyl steht,

$R^{16}$ für Wasserstoff oder $C_1$-$C_4$-Alkyl steht und

$R^{17}$ für $C_1$-$C_4$-Alkyl oder Phenyl steht,

oder

$R^7$ für den Rest -$S(O)_n$-Y steht, worin

n für die Zahlen 0, 1 oder 2 steht und

Y für $C_1$-$C_6$-Alkoxy, $C_2$-$C_6$-Alkenoxy, $C_2$-$C_6$-Alkinoxy, Benzyloxy, Phenoxy oder für Amino, $C_1$-$C_6$-Alkylamino, $C_2$-$C_6$-Alkenylamino, $C_2$-$C_6$-Alkinylamino, Benzylamino, Phenylamino, Di-($C_1$-$C_4$-alkyl)-amino, Cyanamino, Guanidino, Hydroxyamino, $C_1$-$C_6$-Alkoxyamino, Hydrazino, $C_1$-$C_4$-Alkylhydrazino, Phenylhydrazino, $C_1$-$C_4$-Alkylcarbonylhydrazino, $C_1$-$C_4$-Alkoxycarbonylhydrazino, $C_1$-$C_4$-Alkylsulfonylhydrazino oder Phenylsulfonylhydrazino steht,

oder
$R^7$ für den Rest

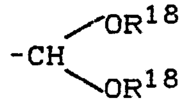

steht, worin die beiden Reste $R^{18}$ einzeln für $C_1$-$C_4$-Alkyl oder zusammen für $C_2$-$C_3$-Alkandiyl stehen,
oder

$R^{19}$

$R^7$ für den Rest $-(O)_p-(A)_q-$ $C = N -R^{20}$ steht, worin
$R^{19}$ für Wasserstoff, $C_1$-$C_4$-Alkyl, $C_1$-$C_4$-Alkoxy oder $C_1$-$C_4$-Alkylamino steht und
$R^{20}$ für Wasserstoff, $C_1$-$C_4$-Alkyl, Phenyl, Hydroxy, für gegebenenfalls durch $C_1$-$C_4$-Alkoxy-carbonyl substituiertes $C_1$-$C_4$-Alkoxy, für Allyloxy, Propargyloxy oder Benzyloxy steht,
A für $C_1$-$C_4$-Alkandiyl steht und
p und q jeweils für die Zahlen 0 oder 1 stehen,
oder
$R^7$ für den Rest

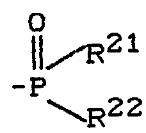

steht, worin
$R^{21}$ für $C_1$-$C_4$-Alkyl oder $C_1$-$C_4$-Alkoxy steht und
$R^{22}$ für $C_1$-$C_4$-Alkoxy steht,
oder
$R^7$ für den Rest $-(CH_2)_m$-Z oder für den Rest

$R^{10}$

-O- $CH-$ $COO-(CH_2)_m$ -Z steht, worin
m für die Zahlen 0, 1 oder 2 steht,
Z für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl, Pyrimidinyl und Pyridazinyl steht und
$R^{10}$ für Wasserstoff oder $C_1$-$C_4$-Alkyl steht.

3. Substituierte Phenoxypyridine der Formel (I) gemäß Anspruch 1, in welcher
$R^1$ für Wasserstoff, Fluor, Chlor, Cyano oder Trifluormethyl steht,
$R^2$ für Wasserstoff, Fluor oder Chlor steht,
$R^3$ für Fluor, Chlor, Cyano, Methyl, Ethyl, n-oder i-Propyl, n-, i-, s-oder t-Butyl, Methoxy, Methylthio, Trifluormethyl, Trifluormethoxy, Trifluormethylthio, Trifluormethylsulfinyl oder Trifluormethylsulfonyl steht,
$R^4$ für Wasserstoff, Fluor oder Chlor steht und
$R^5$ für Wasserstoff, Fluor oder Chlor steht
-mit der Maßgabe, daß wenigstens drei der Substituen $R^1$ bis $R^5$ von Wasserstoff verschieden sind und wenigstens einer der Reste $R^1$ bis $R^5$ von Wasserstoff, Fluor und Chlor verschieden ist -
in welcher weiter
$R^6$ für Wasserstoff, Cyano, Nitro oder Chlor steht und
$R^7$ für Wasserstoff, Chlor, Cyano, Nitro, Hydroxy, Di-($C_1$-$C_2$-alkoxy)-(thio)phosphoryloxy oder für $O^{\ominus}M^{\oplus}$ steht, wobei
$M^{\oplus}$ für ein Ammoniumion, ein $C_1$-$C_3$-Alkylammoniumion, ein Natriumion oder ein Kaliumion steht,
oder
$R^7$ für gegebenenfalls durch Fluor, Chlor, Cyano, Carboxy, Carbamoyl, Acetyl, Phenyl, Amino, $C_1$-$C_3$-Alkylamino, Di-($C_1$-$C_3$-alkyl)-amino, Hydroxy, $C_1$-$C_3$-Alkoxy, $C_1$-$C_3$-Alkylthio, $C_1$-$C_3$-Alkoxycarbonyl und/ oder $C_1$-$C_3$-Alkoxycarbonyl-$C_1$-$C_3$-alkoxy substituiertes $C_1$-$C_3$-Alkyl, für gegebenenfalls durch Fluor, Chlor, Cyano, Di-($C_1$-$C_3$-alkyl)-amino, Hydroxy, $C_1$-$C_3$-Alkylcarbonyl, $C_1$-$C_3$-Alkoxy und/oder $C_1$-$C_3$-Alkylthio substituiertes $C_1$-

$C_3$-Alkoxy, für $C_3$-$C_4$-Alkenyloxy, für $C_3$-$C_4$-Alkinyloxy, für gegebenenfalls durch Fluor, Chlor, Cyano, Carboxy, Carbamoyl, $C_1$-$C_3$-Alkoxy, $C_1$-$C_3$-Alkylthio, $C_1$-$C_3$-Alkoxy-carbonyl substituiertes $C_1$-$C_3$-Alkylthio, für jeweils gegebenenfalls durch Fluor und Chlor substituiertes $C_1$-$C_3$-Alkylsulfinyl oder $C_1$-$C_3$-Alkylsulfonyl, Hydrazino, Phenylhydrazino, Methoxycarbonylhydrazino steht,

oder

$R^7$ für den Rest

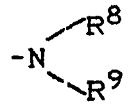

steht, worin

$R^8$ für Wasserstoff oder $C_1$-$C_3$-Alkyl steht und

$R^9$ für Wasserstoff, Hydroxy, Amino, Dimethylamino, Phenylamino, Acetylamino, Benzoylamino, Methylsulfonylamino, Phenylsulfonylamino, Cyano, $C_1$-$C_3$-Alkyl, $C_1$-$C_3$-Alkoxy, $C_1$-$C_3$-Alkoxy-carbonyl, $C_1$-$C_3$-Alkylcarbonyl, Benzoyl, Phenoxycarbonyl, $C_1$-$C_3$-Alkylamino-carbonyl, Phenylamino-carbonyl, $C_1$-$C_3$-Alkoxy-carbonyl-$C_1$-$C_2$-alkyl, Benzyl, $C_1$-$C_3$-Alkylsulfonyl, Phenylsulfonyl oder für gegebenenfalls durch Fluor, Chlor, Brom, Cyano, Nitro, Methyl, Methoxy, Trifluormethyl und/oder Trifluormethoxy substituiertes Phenyl steht,

oder

$R^7$ für den Rest $-O-\overset{R^{10}}{\overset{|}{C}}H-CO-R^{11}$ steht, worin

$R^{10}$ für Wasserstoff oder Methyl steht und

$R^{11}$ für Chlor, Hydroxy, Amino, $C_1$-$C_3$-Alkylamino, Cyanamino, Di-($C_1$-$C_3$-alkyl)-amino, $C_1$-$C_3$-Alkylsulfonylamino, Phenylsulfonylamino, Hydroxyamino, $C_1$-$C_3$-Alkoxyamino, Hydrazino, $C_1$-$C_3$-Alkylsulfonylhydrazino, Phenylsulfonylhydrazino oder für den Rest -Q-$R^{12}$ steht, worin

Q für Sauerstoff oder Schwefel steht und

$R^{12}$ für $C_1$-$C_4$-Alkyl, $C_1$-$C_2$-Alkoxy-ethyl, $C_1$-$C_2$-Alkylthio-ethyl, Trimethylsilylmethyl, Benzyloxyethyl, $C_1$-$C_3$-Alkoxy-carbonyl-$C_1$-$C_2$-alkyl, Benzylthioethyl, Benzyl, Pyrazol-$C_1$-$C_2$-alkyl oder für ein Ammonium-, ein $C_1$-$C_3$-Alkylammoniumion, ein Natrium-oder ein Kalium-äquivalent oder für $C_1$-$C_2$-Alkylamino-carbonyl-$C_1$-$C_2$-alkyl steht,

oder

$R^7$ für den Rest

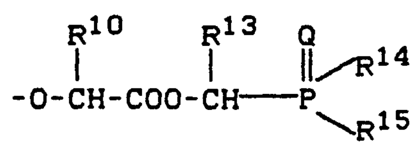

steht, worin

$R^{10}$ für Wasserstoff oder Methyl steht,

$R^{13}$ für Wasserstoff, Methyl, Phenyl, Furyl, Thienyl oder Pyridyl steht,

$R^{14}$ für $C_1$-$C_2$-Alkoxy steht,

$R^{15}$ für $C_1$-$C_2$-Alkoxy steht und

Q für Sauerstoff oder Schwefel steht,

oder

$R^7$ für den Rest $-S(O)_n-Y$ steht, worin

n für die Zahlen 0, 1 oder 2 steht und

Y für $C_1$-$C_4$-Alkyl, $C_1$-$C_2$-Alkoxy-carbonyl-$C_1$-$C_2$-alkyl oder Benzyl steht oder

$R^7$ für den Rest $-(CH_2)_m-Z$ oder

für den rest $-O-\overset{R^{10}}{\overset{|}{C}}H-COO-(CH_2)_m-Z$ steht, worin

m für die Zahlen 0, 1 oder 2 steht,

Z für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl und Dioxolanyl steht und

$R^{10}$ für Wasserstoff oder Methyl steht, oder

$R^{19}$
|
$R^7$ für den Rest -O-$CH_2$- C — = N-$R^{20}$ steht, worin
$R^{19}$ für Wasserstoff, Methyl oder Ethyl steht und
$R^{20}$ für Wasserstoff, Hydroxy, Methyl, Ethyl oder $C_1$-$C_2$-Alkoxycarbonyl-$C_1$-$C_2$-alkyl steht.

4. Verfahren zur Herstellung von substituierten Phenoxypyridinen der Formel (I)

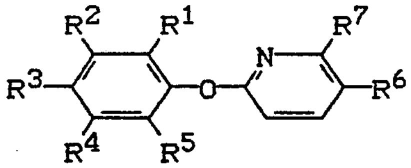

(I)

in welcher
$R^1$ für Wasserstoff, Halogen, Cyano, Nitro oder für einen gegebenenfalls durch Halogen substituierten Rest aus der Reihe Alkyl, Alkoxy, Alkylthio, Alkylsulfinyl und Alkylsulfonyl steht,
$R^2$ für Wasserstoff, Halogen oder Alkyl steht,
$R^3$ für Wasserstoff, Halogen, Cyano, Nitro oder für einen gegebenenfalls durch Halogen substituierten Rest aus der Reihe Alkyl, Alkoxy, Alkylthio, Alkylsulfinyl und Alkylsulfonyl steht,
$R^4$ für Wasserstoff oder Halogen steht und
$R^5$ für Wasserstoff oder Halogen steht
-mit der Maßgabe, daß wenigstens drei der Substituenten $R^1$ bis $R^5$ von Wasserstoff verschieden sind und wenigstens einer der Reste $R^1$ bis $R^5$ von Wasserstoff und Halogen verschieden ist -
in welcher weiter
$R^6$ für Wasserstoff, Cyano, Nitro, Halogen, Carboxy, Carbamoyl oder Alkoxycarbonyl steht und
$R^7$ für Wasserstoff, Halogen, Cyano, Nitro, Formyl, Hydroxy, Trialkylsilyloxy, Alkylaminocarbonyloxy, Dialkoxy(thio)phosphoryloxy, oder für -$O^{\ominus}M^{\oplus}$ steht, wobei
$M^{\oplus}$ für ein Ammoniumion, ein Alkylammoniumion, ein Alkalimetallion oder ein Erdalkalimetallionenäquivalent steht oder
$R^7$ für einen gegebenenfalls durch Halogen, Cyano, Nitro, Carboxy, Carbamoyl, Amino, Phenyl, Alkylcarbonyl, Alkylamino, Dialkylamino, Hydroxy, Alkoxy, Alkylthio, Alkylaminocarbonyl, Alkoxycarbonyl und/oder Alkoxycarbonylalkoxy substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl und Alkylsulfonyl steht oder
$R^7$ für den Rest

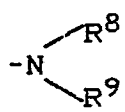

steht, worin
$R^8$ für Wasserstoff oder Alkyl steht und
$R^9$ für Wasserstoff, Hydroxy, Amino, Alkylamino, Dialkylamino, Arylamino, Alkylcarbonylamino, Arylcarbonylamino, Alkylsulfonylamino, gegebenenfalls im Arylteil durch Alkyl substituiertes Arylsulfonylamino, Cyano, Alkyl, Alkoxy, Halogenalkyl, Alkoxycarbonyl, Alkylcarbonyl, Arylcarbonyl, Aryloxycarbonyl, Alkylaminocarbonyl, Arylaminocarbonyl, Alkoxycarbonylalkyl, Aralkyl, Alkylsulfonyl, Arylsulfonyl oder für gegebenenfalls durch Halogen, Cyano, Nitro, Alkyl, Alkoxy, Halogenalkyl und/oder Halogenalkoxy substituiertes Aryl steht, oder

$R^{10}$
|
$R^7$ für den Rest -O- C H — CO-$R^{11}$ steht, worin
$R^{10}$ für Wasserstoff oder Alkyl steht und
$R^{11}$ für Halogen, Hydroxy, Amino, Alkylamino, Cyanamino, Dialkylamino, Alkylsulfonylamino, Arylsulfonylamino, Hydroxyamino, Alkoxyamino, Hydrazino, Alkylsulfonylhydrazino, Arylsulfonylhydrazino oder für den Rest -Q-$R^{12}$ steht, worin
Q für Sauerstoff oder Schwefel steht und
$R^{12}$ für gegebenenfalls durch Halogen substituierte Reste aus der Reihe Alkyl, Alkoxyalkyl, Alkylthioalkyl, Alkylsulfinylalkyl, Alkylsulfonylalkyl, Aryloxyalkyl, Trialkylsilylalkyl, Arylthioalkyl, Aralkoxyalkyl, Alkoxycarbonylalkyl, Alkylaminocarbonylalkyl, Aralkylthioalkyl, Aralkyl, Azolylalkyl oder für ein Ammonium-,

Alkylammonium-, Alkalimetall-oder Erdalkalimetall-äquivalent steht, oder
$R^7$ für den Rest

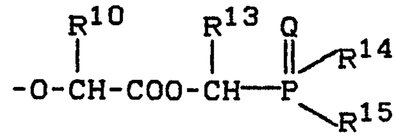

steht,
worin
$R^{10}$ für Wasserstoff oder Alkyl steht,
$R^{13}$ für Wasserstoff, Alkyl, Aryl, Furyl, Thienyl oder Pyridyl steht,
$R^{14}$ für Alkyl oder Alkoxy steht,
$R^{15}$ für Alkoxy steht und
Q für Sauerstoff oder Schwefel steht, oder
$R^7$ für den Rest

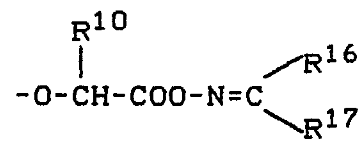

steht, worin
$R^{10}$ für Wasserstoff oder Alkyl steht,
$R^{16}$ für Wasserstoff oder Alkyl steht und
$R^{17}$ für Alkyl oder Aryl steht, oder
$R^7$ für den Rest $-S(O)_n-Y$ steht, worin
n für die Zahlen 0, 1 oder 2 steht und
Y für Alkoxy, Alkenoxy, Alkinoxy, Aralkoxy, Aryloxy oder für Amino, Alkylamino, Alkenylamino, Alkinylamino, Aralkylamino, Arylamino, Dialkylamino, Cyanamino, Guanidino, Hydroxyamino, Alkoxyamino, Hydrazino, Alkylhydrazino, Arylhydrazino, Alkylcarbonylhydrazino, Alkoxycarbonylhydrazino, Alkylsulfonylhydrazino oder Arylsulfonylhydrazino steht, oder
$R^7$ für den Rest

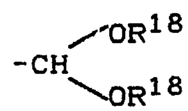

steht, worin die beiden Reste $R^{18}$ einzeln für Alkyl oder zusammen für Alkandiyl ("Alkylen") stehen, oder
$R^7$ für den Rest

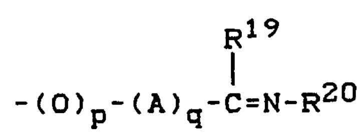

steht, worin
A für Alkandiyl (Alkylen) steht,
p und q jeweils für die Zahlen 0 oder 1 stehen,
$R^{19}$ für Wasserstoff, Alkyl, Alkoxy oder Alkylamino steht und
$R^{20}$ für Wasserstoff, Alkyl, Aryl, Hydroxy, für gegebenenfalls durch Alkoxycarbonyl substituiertes Alkoxy, für Alkenyloxy, Alkinyloxy oder Aralkoxy steht, oder
$R^7$ für den Rest

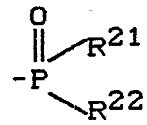

steht, worin
$R^{21}$ für Alkyl oder Alkoxy steht und
$R^{22}$ für Alkoxy steht, oder
$R^7$ für den Rest $-(CH_2)_m-Z$ oder für den Rest

$-O-\overset{R^{10}}{\overset{|}{C}}H-COO-(CH_2)_m-Z$ steht, worin
m für die Zahlen 0, 1 oder 2 steht,
$R^{10}$ für Wasserstoff oder Alkyl steht, und
Z für einen gegebenenfalls durch Halogen und/ oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiozolyl, Dioxolanyl, Perhydropyrrolyl, Oxoperhydropyrrolyl, Pyridinyl, Pyrimidinyl und Pyridazinyl steht,
dadurch gekennzeichnet, daß man

(a) Phenole der Formel (II)

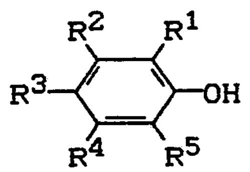
(II)

in welcher
$R^1$, $R^2$, $R^3$, $R^4$ und $R^5$ die oben angegebenen Bedeutungen haben,
oder Alkalimetallsalze von Phenolen der Formel (II) mit Halogenpyridinen der Formel (III)

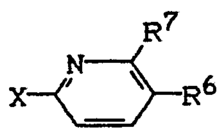
(III)

in welcher
$R^6$ und $R^7$ die oben angegebenen Bedeutungen haben und
X für Halogen steht,
gegebenenfalls in Gegenwart von Säureakzeptoren und gegebenenfalls in Gegenwart von Verdünnungsmitteln umsetzt
oder daß man

(b) für den Fall, daß $R^7$ für Hydroxy steht und die Reste $R^1$ bis $R^6$ die oben angegebenen Bedeutungen haben, wenn man Verbindungen der Formel (I), in welcher $R^7$ für Amino steht und die Reste $R^1$ bis $R^6$ die oben angegebenen Bedeutungen haben, mit einem Alkalinitrit in Gegenwart einer wäßrigen Mineralsäure umsetzt, oder daß man

(c) für den Fall, daß $R^7$ für Carboxyalkoxy ($-O-CHR^{10}-COOH$) steht und die Reste $R^1$ bis $R^6$ die oben angegebene Bedeutung haben, wenn man Verbindungen der Formel (I), in welcher $R^7$ für Alkoxycarbonylalkoxy steht und die Reste $R^1$ bis $R^6$ die oben angegebenen Bedeutungen haben,
mit einem Alkalihydroxid in Gegenwart von Wasser und gegebenenfalls in Gegenwart eines organischen Lösungsmittels umsetzt und dann - gegebenenfalls nach Einengen - mit einer Mineralsäure ansäuert, oder daß man

(d) für den Fall, daß $R^7$ für Chlorcarbonylalkoxy steht und die Reste $R^1$ bis $R^6$ die oben angegebene Bedeutung haben, wenn man Verbindungen der Formel (I), in welcher $R^7$ für Carboxyalkoxy steht und die Reste $R^1$ bis $R^6$ die oben angegebenen Bedeutungen haben,
mit einem Halogenierungsmittel gegebenenfalls in Gegenwart eines Verdünnungsmittels umsetzt, oder daß man

(e) für den Fall, daß $R^7$ für den Rest

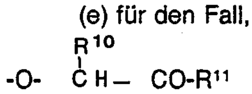

oder für den Rest 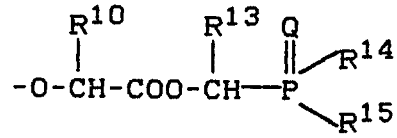

oder für den Rest 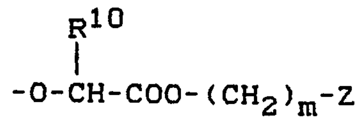

oder für den Rest 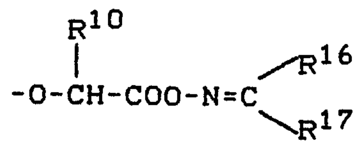

steht, worin $R^{10}$, $R^{11}$, $R^{13}$, $R^{14}$, $R^{15}$, $R^{16}$, $R^{17}$, Z, Q und m die oben angegebenen Bedeutungen haben und weiter die Reste $R^1$ bis $R^6$ die oben angegebenen Bedeutungen haben,
wenn man Verbindungen der Formel (I), in welcher $R^7$ für Chlorcarbonylalkoxy steht und die Reste $R^1$ bis $R^6$ die oben angegebenen Bedeutungen haben
-Chlorcarbonylalkoxy steht in diesem Fall für den
Rest 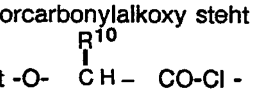
mit Verbindungen der Formel H - $R^{11}$

bzw. der Formel 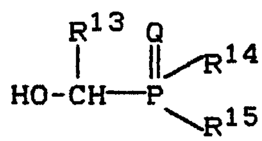

bzw. der Formel $HO-(CH_2)_m-Z$

bzw. der Formel 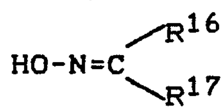

worin $R^{11}$, $R^{13}$, $R^{14}$, $R^{15}$, $R^{16}$, $R^{17}$, Z, Q und m die oben angegebenen Bedeutungen haben, gegebenenfalls in Gegenwart eines Säureakzeptors und gegebenenfalls in Gegenwart eines Verdünnungsmittels umsetzt, oder daß man

(f) für den Fall, daß $R^7$ für den Rest

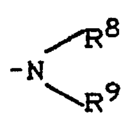

steht, worin
$R^8$ die oben angegebene Bedeutung hat und
$R^9$ für Alkoxycarbonyl, Alkylcarbonyl, Arylcarbonyl, Aryloxycarbonyl, Alkylaminocarbonyl, Arylaminocarbonyl, Alkylsulfonyl oder Arylsulfonyl steht
und die Reste $R^1$ bis $R^6$ die oben angegebenen Bedeutungen haben,
wenn man Verbindungen der Formel (I), in welcher $R^7$ für den Rest -NH-$R^8$ steht, worin $R^8$ die oben angegebene Bedeutung hat, und die Rest $R^1$ bis $R^6$ die oben angegebenen Bedeutungen haben,

mit Alkoxycarbonyl-, Alkylcarbonyl-, Arylcarbonyl-, Aryloxycarbonyl-, Alkylsulfonyl-oder Arylsulfonyl-halogeniden (insbesondere -chloriden) bzw. den entsprechenden Anhydriden bzw. mit Alkyl-oder Aryl-isocyanaten, gegebenenfalls in Gegenwart eines Säureakzeptors und gegebenenfalls in Gegenwart eines Verdünnungsmittels umsetzt, oder daß man

(g) für den Fall, daß $R^7$ für Alkylsulfinyl oder Alkylsulfonyl steht und die Reste $R^1$ bis $R^6$ die oben angegebenen Bedeutungen haben,
wenn man Verbindungen der Formel (I), in welcher $R^7$ für Alkylthio steht und die Reste $R^1$ bis $R^6$ die oben angegebenen Bedeutungen haben,
mit einem Oxidationsmittel, gegebenenfalls in Gegenwart eines Katalysators und gegebenenfalls in Gegenwart eines Verdünnungsmittels umsetzt, oder daß man

(h) für den Fall, daß $R^7$ für Alkoxy steht und die Reste $R^1$ bis $R^6$ die oben angegebenen Bedeutungen haben,
wenn man Verbindungen der Formel (I), in welcher $R^7$ für Halogen steht und die Reste $R^1$ bis $R^6$ die oben angegebenen Bedeutungen haben,
mit Alkanolen und/oder Alkalimetallsalzen von Alkanolen umsetzt, oder daß man

(i) für den Fall, daß $R^7$ für Halogen steht und die Reste $R^1$ bis $R^6$ die oben angegebenen Bedeutungen haben,
wenn man Verbindungen der Formel (I), in welcher $R^7$ für Amino steht und die Reste $R^1$ bis $R^6$ die oben angegebenen Bedeutungen haben,
mit einem Alkalimetallnitrit und einem Hydrogen-oder Metall-halogenid bzw. Metallhalogenid-Komplex, in Gegenwart einer Säure, gegebenenfalls in Gegenwart eines Katalysators und in Gegenwart eines Verdünnungsmittels umsetzt, oder daß man

(j) für den Fall, daß $R^7$ für den Rest $-O-\overset{R^{10}}{\overset{|}{C}}H-COOR^{12}$ steht, worin
$R^{10}$ für Wasserstoff oder Alkyl steht und
$R^{12}$ für Trialkylsilylalkyl steht,
und die Reste $R^1$ bis $R^6$ die oben angegebenen Bedeutungen haben,
wenn man Verbindungen der Formel (I), in welcher
$R^7$ für den Rest $-O-\overset{R^{10}}{\overset{|}{C}}H-COOH$ steht, worin $R^{10}$ die oben angegebenen Bedeutungen hat,
mit Trialkylsilylalkylhalogeniden, gegebenenfalls in Gegenwart eines Säureakzeptors und gegebenenfalls in Gegenwart eines Verdünnungsmittels umsetzt.

5. Herbizide und pflanzenwuchsregulierende Mittel, gekennzeichnt durch einen Gehalt an mindestens einem substituierten Phenoxypyridin der Formel (I) gemäß den Ansprüchen 1 bis 4.

6. Verfahren zur Bekämpfung von Unkräutern, dadurch gekennzeichnet, daß man substituierte Phenoxypyridine der Formel (I) gemäß den Ansprüchen 1 bis 4 auf die Unkräuter und/oder ihren Lebensraum einwirken läßt.

7. Verwendung von substituierten Phenoxypyridinen der Formel (I) gemäß den Ansprüchen 1 bis 4 zur Bekämpfung von Unkräutern und/oder als Pflanzenwachstumsregulaturen.

8. Verfahren zur Herstellung von herbiziden und/oder pflanzenwuchsregulierenden Mitteln, dadurch gekennzeichnet, daß man substituierte Phenoxypyridine der Formel (I) gemäß den Ansprüchen 1 bis 4 mit Streckmitteln und/oder oberflächenaktiven Substanzen vermischt.

9. Phenole der Formel (IIa)

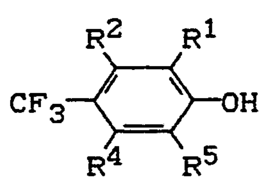

(IIa)

in welcher
$R^1$, $R^2$, $R^4$ und $R^5$ unabhängig voneinander für Wasserstoff, Fluor oder Chlor stehen, wobei wenigstens einer dieser Substituenten für Wasserstoff steht, wenigstens ein weiterer Substituent für Fluor steht sowie wenigstens ein dritter dieser Substituenten für Fluor oder Chlor steht.

10. Verfahren zur Herstellung von Phenolen der Formel (IIa)

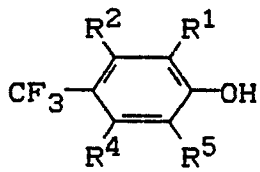

(IIa)

in welcher
$R^1$, $R^2$, $R^4$ und $R^5$ unabhängig voneinander für Wasserstoff, Fluor oder Chlor stehen, wobei wenigstens einer dieser Substituenten für Wasserstoff steht, wenigstens ein weiterer Substituent für Fluor steht sowie wenigstens ein dritter dieser Substituenten für Fluor oder Chlor steht,
dadurch gekennzeichnet, daß man Halogenbenzol-Derivate der Formel (IV)

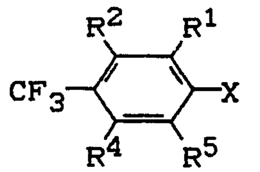

(IIa)

in welcher
$R^1$, $R^2$, $R^4$ und $R^5$ die oben angegebene Bedeutung haben und
X für Fluor oder Chlor steht,
mit Alkalimetall-und/oder Erdalkalimetall-hydroxiden gegebenenfalls in Gegenwart eines Phasentransferkatalysators und in Gegenwart von Verdünnungsmitteln umsetzt.